# EUROPEAN PATENT APPLICATION

(11) **EP 4 234 032 A2**
(43) Date of publication of application: **30.08.2023**
(21) Application number: 23169770.7
(22) Date of filing: 24.09.2015
(51) Int. Cl.: A61P 35/00

(54) **INDUCTION MEDIUM AND METHODS FOR STEM CELL CULTURE AND THERAPY**

(30) Priority: 01.10.2014 US 201414504399
(62) Divisional of application: 15847382.7
(71) Applicant: SanBio, Inc., Mountain View, CA 94041 (US)
(72) Inventor: BETANCOURT, Aline, California, 92037 (US)
(74) Representative: J A Kemp LLP

(57) **Abstract**

Novel MSC stem-cell culture and therapy methods and culture medium compositions for the purpose of inducing, activating, or priming discrete uniform cell phenotypes to selectively promote or suppress inflammation and immunity, yielding polarized, primed, activated, or induced cells used in cell-based therapy.

## Description

### STATEMENT AS TO FEDERALLY SPONSORED RESEARCH

This invention was made with United States government support under NIH 1R43AR061902-01, and IP20RR20152-01; and Department of Defense OC073102 and OC110218. The government has certain rights in the invention.

### SUMMARY OF THE INVENTION

The invention provides novel stem-cell culture and therapy methods and culture medium compositions for the purpose of inducing, activating, or priming discrete uniform cell phenotypes to selectively promote or suppress inflammation and immunity, providing significant advantages over known culture medium and methods, used in cell-based therapy. The invention can be used to provide more uniform and predictable ex-vivo expanded and induced, primed, or activated populations of mesenchymal stem cells (MSCs), which can be used for cell-based therapy. There is a long-felt need in the art for an improved method to provide a uniform and efficacious large number of stem cells required for cell-based therapy. An advantage of the various embodiments of the invention is that they can be used to induce, activate or prime cultures of mesenchymal stem cells into uniform and discrete phenotypes that behave in a predictable manner upon introduction into a patient.

In certain embodiments, disclosed herein is an induction medium for creating an immunologically polarized mesenchymal stem cell population from an unstimulated mesenchymal stem cell population, the induction medium comprising: a Toll-like receptor 3 (TLR3) ligand, erythropoietin, and 0.5-2% oxygen or a hypoxia mimetic, wherein the immunologically polarized mesenchymal stem cell population possess anti-inflammatory characteristics marked by expression of anti-inflammatory or immunosuppressive mediators. In certain embodiments, the Toll-like receptor 3 (TLR3) ligand is poly(T:C). In certain embodiments, the Toll-like receptor 3 (TLR3) ligand is poly(A:U). In certain embodiments, erythropoietin is present at a concentration of less than 10 ng/mL. In certain embodiments, the hypoxia mimetic is cobalt chloride. In certain embodiments, the cobalt chloride is present at a concentration of between 5 µM and 500 µM. In certain embodiments, the induction medium further comprises interleukin 4 (IL-4). In certain embodiments, the induction medium further comprises interleukin 13 (IL-13). In certain embodiments, the induction medium does not comprise serum of human or animal origin. In certain embodiments, the induction medium is a concentrated solution. In certain embodiments, disclosed herein is a mesenchymal stem cell population treated with the induction medium. In certain embodiments, disclosed herein is a human mesenchymal stem cell population treated with the induction medium. In certain embodiments, disclosed herein is a canine, feline, or equine mesenchymal stem cell population treated with the induction medium. In certain embodiments, disclosed herein is a mesenchymal stem cell population treated with the induction medium. In certain embodiments, disclosed herein is a mesenchymal stem cell population treated with the induction medium, wherein the cells are marked by increased expression of *CXCL9* mRNA compared to an unstimulated mesenchymal stem cell population. In certain embodiments, disclosed herein is a mesenchymal stem cell population treated with the induction medium, wherein the cells are marked by increased expression of *OAS1* mRNA compared to an unstimulated mesenchymal stem cell population. In certain embodiments, disclosed herein is a mesenchymal stem cell population treated with the induction medium, wherein the cells are marked by increased expression of *ISG15* mRNA compared to an unstimulated mesenchymal stem cell population. In certain embodiments, disclosed herein is a composition for the treatment of a disease comprising a mesenchymal stem cell population treated with the induction medium, wherein the disease is an inflammatory or autoimmune disorder. In certain embodiments, the inflammatory or autoimmune disorder is rheumatoid arthritis. In certain embodiments, the inflammatory or autoimmune disorder is an inflammatory bowel disease. In certain embodiments, the inflammatory or autoimmune disorder is Acute Optic Neuritis. In certain embodiments, the inflammatory or autoimmune disorder is Krabbe disease. In certain embodiments, the inflammatory or autoimmune disorder is Diabetic Retinopathy. In certain embodiments, the inflammatory or autoimmune disorder is Crohn's Disease. In certain embodiments, the inflammatory or autoimmune disorder is Acute Lung Injury.

In certain embodiments, disclosed herein is an induction medium for creating an immunologically polarized mesenchymal stem cell population from an unstimulated mesenchymal stem cell population, the induction medium comprising: a Toll-like receptor 4 (TLR4) ligand, erythropoietin, and 0.5-2% oxygen or a hypoxia mimetic, wherein the immunologically polarized mesenchymal stem cell population possess pro-inflammatory characteristics marked by expression of pro-inflammatory mediators. In certain embodiments, the Toll-like receptor 4 (TLR4) ligand is lipopolysaccharide (LPS). In certain embodiments, the Toll-like receptor 4 (TLR4)ligand is an aminoalkyl glucosaminide 4-phosphate. In certain embodiments, erythropoietin is present at a concentration of less than 10 ng/mL. In certain embodiments, the hypoxia mimetic is cobalt chloride. In certain embodiments, the cobalt chloride is present at a concentration of between 5 µM and 500 µM. In certain embodiments, the induction medium further comprises an interferon. In certain embodiments, the induction medium further comprises tumor necrosis factor alpha (TNFα). In certain embodiments, the induction medium does not comprise serum of human or animal origin. In certain embodiments, the induction medium is a concentrated solution. In certain embodiments, disclosed herein is a mesenchymal stem cell population treated with the induction medium. In certain embodiments, disclosed herein is a human mesenchymal stem cell population treated with the induction medium. In certain embodiments, disclosed herein is a canine, feline or equine mesenchymal stem cell population treated with the induction medium. In certain embodiments, disclosed herein is a mesenchymal stem cell population treated with the induction medium, wherein the mesenchymal stem cells were derived from a pluripotent stem cell. In certain embodiments, disclosed herein is a mesenchymal stem cell population treated with the induction medium, wherein the cells are marked by increased expression of *TNFSF10 (TRAIL)* mRNA compared to an unstimulated mesenchymal stem cell population. In certain embodiments, disclosed herein is a composition for the treatment of a disease comprising a mesenchymal stem cell population treated with the induction medium, wherein the disease is a cancer. In certain embodiments, the cancer is ovarian cancer. In certain embodiments, the cancer is uveal melanoma. In certain embodiments, disclosed herein is a composition for the treatment of a disease comprising a mesenchymal stem cell population treated with the induction medium, wherein the disease is a viral condition. In certain embodiments, disclosed herein is a composition for the treatment of a disease comprising a mesenchymal stem cell population treated with the induction medium of, wherein the disease is a bacterial infection.

In certain embodiments, disclosed herein is an induction medium for creating an immunologically polarized mesenchymal stem cell population from an unstimulated mesenchymal stem cell population, the induction medium comprising: Poly(I:C) at a concentration of between 0.1 µg/mL and 100 µg/mL, erythropoietin at a concentration of less than 10 ng/mL, and cobalt chloride at a concentration of between 5 µM and 500 µM, wherein the immunologically polarized mesenchymal stem cell population possess anti-inflammatory characteristics and is marked by increased expression of *CXCL9, OAS1* and *ISG15* mRNA compared to an unstimulated mesenchymal stem cell population.

In certain embodiments, disclosed herein is an induction medium for creating an immunologically polarized mesenchymal stem cell population from an unstimulated mesenchymal stem cell population, the induction medium comprising: LPS at a concentration of between 0.1 ng/mL and 1 µg/mL, erythropoietin at a concentration of less than 10 ng/mL, and cobalt chloride at a concentration of between 5 µM and 500 µM, wherein the immunologically polarized mesenchymal stem cell population possess pro-inflammatory characteristics and is marked by increased expression of *TNFSF10 (TRAIL)* compared to an unstimulated mesenchymal stem cell population.

In certain embodiments, disclosed herein is a method for creating an immunologically polarized mesenchymal stem cell population from an unstimulated mesenchymal stem cell population, the method comprising: contacting an unstimulated mesenchymal stem cell population with a composition comprising; a Toll-like receptor 3 (TLR3) ligand, erythropoietin, and hypoxia or a hypoxia mimetic, wherein the immunologically polarized mesenchymal stem cell population possess anti-inflammatory characteristics marked by expression of anti-inflammatory or immunosuppressive mediators. In certain embodiments, the TLR3 ligand is poly(I:C). In certain embodiments, the TLR3 ligand is poly(A:U). In certain embodiments, erythropoietin is present at a concentration of less than 10 ng/mL. In certain embodiments, the hypoxia mimetic is cobalt chloride. In certain embodiments, the cobalt chloride is present at a concentration of between 5 µM and 500 µM. In certain embodiments, the composition further comprises interleukin 4 (IL-4). In certain embodiments, the composition further comprises interleukin 13 (IL-13). In certain embodiments, the composition does not comprise serum of human or animal origin. In certain embodiments, the composition is a concentrated solution. In certain embodiments, the unstimulated mesenchymal stem cell population is contacted with a Toll-like receptor 3 ligand, erythropoietin, and hypoxia or a hypoxia mimetic simultaneously. In certain embodiments, the composition is in contact with the unstimulated mesenchymal stem cell population for at least 30 minutes, but less than 8 hours. In certain embodiments, the method further comprises monitoring expression of *CXCL9* at either the RNA or protein level. In certain embodiments, the method, further comprises monitoring expression of *OAS1* at either the RNA or protein level. In certain embodiments, the method further comprises monitoring expression of *ISG15* at either the RNA or protein level. In certain embodiments, provided herein is a mesenchymal stem cell population treated by the method. In certain embodiments, provided herein is a human mesenchymal stem cell population treated by the method. In certain embodiments, provided herein is a canine, feline or equine mesenchymal stem cell population treated by the method. In certain embodiments, provided herein is a mesenchymal stem cell population treated by the method, wherein the mesenchymal stem cells were derived from a pluripotent stem cell. In certain embodiments, provided herein is a mesenchymal stem cell population treated by the method, wherein the cells are marked by increased expression of *CXCL9* mRNA compared to an unstimulated mesenchymal stem cell population. In certain embodiments, provided herein is a mesenchymal stem cell population treated by the method, wherein the cells are marked by increased expression of *OAS1* mRNA compared to an unstimulated mesenchymal stem cell population. In certain embodiments, provided herein is a mesenchymal stem cell population treated by the method, wherein the cells are marked by increased expression of *ISG15* mRNA compared to an unstimulated mesenchymal stem cell population. In certain embodiments, provided herein is a composition for the treatment of a disease comprising a mesenchymal stem cell population treated by the method, wherein the disease is an inflammatory or autoimmune disorder. In certain embodiments, the inflammatory or autoimmune disorder is rheumatoid arthritis. In certain embodiments, the inflammatory or autoimmune disorder is an inflammatory bowel disease. In certain embodiments, the inflammatory or autoimmune disorder is Acute Optic Neuritis. In certain embodiments, the inflammatory or autoimmune disorder is Krabbe disease. In certain embodiments, the inflammatory or autoimmune disorder is Diabetic Retinopathy. In certain embodiments, the inflammatory or autoimmune disorder is Crohn's Disease. In certain embodiments, the inflammatory or autoimmune disorder is Acute Lung Injury.

In certain embodiments, disclosed herein is a method for creating an immunologically polarized mesenchymal stem cell population from an unstimulated mesenchymal stem cell population, the method comprising: contacting an unstimulated mesenchymal stem cell population with a composition comprising; a Toll-like receptor 4 (TLR4) ligand, erythropoietin, and hypoxia or a hypoxia mimetic, wherein the immunologically polarized mesenchymal stem cell population possess pro-inflammatory characteristics marked by expression of pro-inflammatory or immunosuppressive mediators. In certain embodiments, the TLR4 ligand is lipopolysaccharide (LPS). In certain embodiments, TLR4 ligand is an aminoalkyl glucosaminide 4-phosphate. In certain embodiments, erythropoietin is present at a concentration of 1 ng/mL. In certain embodiments, the hypoxia mimetic is cobalt chloride. In certain embodiments, the cobalt chloride is present at a concentration of between 5 µM and 500 µM. In certain embodiments, the composition further comprises an interferon. In certain embodiments, the composition further comprises tumor necrosis factor alpha (TNFα). In certain embodiments, the composition does not comprise serum of human or animal origin. In certain embodiments, the composition is a concentrated solution. In certain embodiments, the unstimulated mesenchymal stem cell population is contacted with a Toll-like receptor 4 ligand, erythropoietin, and hypoxia or a hypoxia mimetic simultaneously. In certain embodiments, the composition is in contact with the unstimulated mesenchymal stem cell population for at least 30 minutes, but less than 8 hours. In certain embodiments, the method further comprises monitoring expression of *TNFSF10 (TRAIL)* at either the RNA or protein level. In certain embodiments, provided herein is a mesenchymal stem cell population treated by the method. In certain embodiments, provided herein is a human mesenchymal stem cell population treated by the method. In certain embodiments, provided herein is a canine, feline or equine mesenchymal stem cell population treated by the method. In certain embodiments, provided herein is a mesenchymal stem cell population treated by the method, wherein the mesenchymal stem cells were derived from a pluripotent stem cell. In certain embodiments, provided herein is a mesenchymal stem cell population treated by the method, wherein the cells are marked by increased expression of *TNFSF 10 (TRAIL)* mRNA compared to an unstimulated mesenchymal stem cell population. In certain embodiments, provided herein is a composition for the treatment of a disease comprising a mesenchymal stem cell population treated by the method, wherein the disease is a cancer. In certain embodiments, the cancer is ovarian cancer. In certain embodiments, the cancer is uveal melanoma. In certain embodiments, provided herein is a composition for the treatment of a disease comprising a mesenchymal stem cell population treated by the method, wherein the disease is a viral condition. In certain embodiments, provided herein is a composition for the treatment of a disease comprising a mesenchymal stem cell population treated by the method, wherein the disease is a bacterial infection.

### UTILITY OF INVENTION

There is a need for improved therapeutic methods and improved cell-culture methods and medium for inducing, activating, or priming uniform populations of MSCs - stem cells, mesenchymal stem cells, marrow stromal cells, multipotent stromal cells, multipotent stem cells - derived from various adult tissues. Clinical applications of MSCs require reproducible cell culture methods and cell expansion methods that provide adequate numbers of cells of suitable quality and consistent therapeutic benefits. Different culture medium and methods have had varying degrees of success. There remains a need for further improvements to MSC culture medium and methods that ensures expanded yields of primed, activated, or induced cells used in cell-based therapy having safe and consistently reproducible therapeutic effects.

The potency or therapeutic benefit of induced, activated, or primed MSC over un-induced conventional MSCs has been demonstrated in pre-clinical models of disease. Anti-inflammatory induced-MSC therapy alleviated pain and inflammation in models of painful diabetic peripheral neuropathy, rheumatoid arthritis, inflammatory bowel disease, and acute lung injury in a significantly improved manner over conventional MSC therapy. Additionally, anti-inflammatory induced-MSC therapy improved clinical scores, gait, and motor function in a pre-clinical model of multiple sclerosis (EAE) and Krabbe's disease. In a murine immune competent ovarian cancer model, the pro-immune anti-tumor induced-MSC cell-based immunotherapy led to attenuation of tumor growth and spread, whereas conventional MSC therapy promoted tumor growth and spread.

### SCIENTIFIC BASIS OF INVENTION

The stimulation of specific Toll-like receptors (TLRs) affects the immune modulating responses of MSCs. Toll-like receptors recognize "danger" signals, and their activation leads to profound cellular and systemic responses that mobilize innate and adaptive host immune cells. The danger signals that trigger TLRs are released following most tissue pathologies. Since danger signals recruit immune cells to sites of injury, the Inventor reasoned that MSCs might be recruited in a similar way. The Inventor observed that MSCs express several TLRs (*e*.*g*., TLR3 and TLR4, known in the art), and that their migration, invasion, and secretion of immune modulating factors is drastically affected by specific TLR-agonist engagement. In particular, the Inventor observed diverse consequences to the MSCs following stimulation of TLR3 when compared to TLR4 by a low-level, short-term TLR-priming protocol. Based on these findings, the Inventor proposed a new paradigm for MSCs that took its cue from the monocyte literature. Specifically, that MSCs can be polarized (induced, activated, or primed) by downstream TLR signaling into two homogenously acting phenotypes classified as MSC1 and MSC2. TLR4-primed MSCs, or MSC1, mostly express pro-immunity inflammatory mediators, while TLR3-primed MSCs, or MSC2, mostly express anti-inflammatory or immunosuppressive ones. Additionally, the Inventor demonstrated that allogeneic (non-self) co-cultures of TLR-primed MSCs with peripheral blood mononuclear cells (PBMCs) predictably lead to suppressed T-lymphocyte activation following MSC2 co-culture, and permissive T-lymphocyte activation in co-culture with MSC1. The induction of MSCs into the pro-immune MSC1 phenotype by TLR4 activation or into the anti-inflammatory MSC2 phenotype by TLR3 activation ensures uniform and defined cells that solves an industry hurdle by providing defined and predictable cells for use in cell-based therapy applications.

Erythropoietin, also known as EPO, is a glycoprotein hormone that controls erythropoiesis, or red blood cell production. It is a cytokine or cell-signaling molecule for erythrocyte (red blood cell) precursors in the bone marrow. Human EPO has a molecular weight of 34 kDa and is also called hematopoietin or hemopoietin. EPO is produced by interstitial fibroblasts in the kidney in close association with peritubular capillary and tubular epithelial tubule and in perisinusoidal cells in the liver. While liver production predominates early in development (fetal and perinatal period), the kidney is the predominant EPO production site in adults. In addition to erythropoiesis, erythropoietin also has other known biological functions. For example, it plays an important role in the brain's response to neuronal injury by providing a pro-survival anti-apoptosis (programmed cell death) signal. EPO is also involved in the wound healing process. Synthetic erythropoietin is also produced by recombinant DNA technology in cell culture. Additionally, several different pharmaceutical EPO-like agents are available with a variety of glycosylation patterns, and are collectively called erythropoiesis-stimulating agents (ESA). EPO is used in this invention as a means to prevent premature cell death and prolong survival of the yielded primed, activated, or induced cells used in cell-based therapy.

Consistent oxygen supply is an important factor influencing all major aspects of cell biology including survival, proliferation, differentiation, and migration. Typically, mammalian cells (not stem cells) require a consistent supply of oxygen to maintain a robust energy production, and to preserve normal cell function and cell survival. By contrast, mammalian stem cells seem to thrive and persist in the hypoxic environment (with oxygen tension ranging from 0.5% to 7%) of the bone marrow. Several studies have shown that the hypoxic environment is required for maintaining the proliferation and self-renewal capability of the stem cells in the bone marrow. Particularly, the effects of reduced oxygen tension even after short-term culture of MSCs has been described as a general method of improving their engraftment capability in cell-based therapies. A hypoxic environment is used in this invention as a means to maintain the self-renewal and proliferative potential of the yielded primed, activated, or induced cells used in cell-based therapy.

### DEFINITIONS AND PREFERRED VALUES

For clear understanding, terms are defined and preferred values are stated here, and also throughout the text where necessary.

The term "cancer" means any disease caused by an uncontrolled division of cells in a part of the body. Cancers include but are not limited to leukemia, lymphoma, melanoma, carcinoma, sarcoma, adenoma or any other malignant tumor or neoplasm caused by a genetic, environmental or stochastic mechanism.

The term "stem cell" means a cell which is capable of giving rise to multiple different types of cells. The term "mesenchymal stem cell" or "MSC" means a stem cell originally derived from the mesenchyme. The term refers to a cell which is capable of differentiating into at least two or more of an osteoblast, a chondrocyte, an adipocyte, or a myocyte. MSCs may be isolated from any type of adult tissue. Typically MSCs are isolated from bone marrow, adipose tissue, umbilical cord, or peripheral blood. In a preferred aspect of the invention, MSCs are obtained from bone marrow or lipoaspirates, themselves obtained from adipose tissue.

The term "multipotent" and alternative term "pluripotent" mean a cell which is capable of giving rise to multiple types of cells of different tissue lineages. The term "multipotent" or "pluripotent" also encompasses induced multipotent stem cells or induced pluripotent stem cells, or cells that have been induced to a pluripotent stage using any chemical or genetic means. In certain embodiments, the multipotent or pluripotent stem cells of the disclosure are mesenchymal stem cells.

The cells of this disclosure are derived from any cell of any mammalian species including human, primate, dog, cat, horse, cow, goat, sheep, and pig. The cell can be a primary cell or an immortalized cell line.

The term "cellular therapy" or "cell-based therapy" means the transplantation of human or animal cells to prevent, treat, or ameliorate one or more symptoms associated with a disease or disorder, such as, but not limited to, the replacement or repair of damaged tissues or organs, the modulation of immune reactions and the reduction of inflammatory symptoms and cancers.

The term "subject" refers to an animal, preferably a mammal including non-primates (*e*.*g*., a cow, pig, horse, cat, dog, rat, or mouse) or a primate (*e*.*g*., a monkey, or a human). In a preferred embodiment, the subject is a human.

The terms "treat", "treatment", and "treating" when used directly in reference to a patient or subject mean the amelioration of one or more symptoms associated with a disorder including, but not limited to any cancer, any tumor or neoplasm, an inflammatory disorder, an autoimmune disease or an immunologically mediated disease including rejection of transplanted organs and tissues, where the amelioration results from the administration of the immunomodulatory cells yielded by the invention, or a pharmaceutical composition comprising immunomodulatory cells yielded by the invention, to a subject in need of such treatment.

The term "unstimulated" refers to a cell population that has been untreated, unpolarized or uninduced by the methods of this disclosure. Primary isolated mesenchymal stem cells that are fresh or frozen are considered unstimulated. Cells that have been previously treated with a compound or composition that lacks at least one of a toll-like receptor ligand, erythropoietin, hypoxia or a hypoxia mimetic are considered unstimulated.

The terms "repair" and "repairing" when used directly in reference to damaged tissues means the amelioration of such damage by both direct mechanisms such as the regeneration of damaged tissues, as well as through indirect mechanisms, *e*.*g*., reducing inflammation thereby enabling tissue formation.

"Allogenic" means from different individuals of the same species. When individuals possess genes that differ at one or more loci, they are said to be allogenic. In contrast, "autologous" means from the same individual.

The term "immune disease" refers to a condition in a subject characterized by cellular, tissue and/or organ injury caused by an immunological reaction of the subject.

The term "autoimmune disorder" refers to a condition in a subject characterized by cellular, tissue and/or organ injury caused by an immunological reaction of the subject to its own cells, tissues, and/or organs. Illustrative, non-limiting examples of autoimmune diseases which can be treated with the immunomodulatory cells yielded by the invention include alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue- dermatitis, chronic fatigue immune dysfunction syndrome (CF1DS), chronic inflammatory demyelinating polyneuropathy, Churg-Strauss syndrome, cicatrical pemphigoid, CREST syndrome, cold agglutinin disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, Meniere's disease, mixed connective tissue disease, multiple sclerosis, type 1 or immune-mediated diabetes mellitus, myasthenia gravis, pemphigus vulgaris, pernicious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, sarcoidosis, scleroderma, progressive systemic sclerosis, Sjogren's syndrome, Good pasture's syndrome, stiff-man syndrome, systemic lupus erythematosus, lupus erythematosus, takayasu arteritis, temporal arteristis/giant cell arteritis, ulcerative colitis, uveitis, vasculitides such as dermatitis herpetiformis vasculitis, vitiligo, Wegener's granulomatosis, Anti-Glomerular Basement Membrane Disease, Antiphospholipid Syndrome, Autoimmune Diseases of the Nervous System, Familial Mediterranean Fever, Lambert-Eaton Myasthenic Syndrome, Sympathetic Ophthalmia, Polyendocrinopathies, Psoriasis, *etc.*

"Immune disorders" include autoimmune diseases and immunologically mediated inflammatory diseases.

"Immune mediated inflammatory disease" means any disease characterized by chronic or acute inflammation, resulting from, associated with, or triggered by, a dysregulation of the normal immune response; *e*.*g*., Crohn's disease, type 1 diabetes mellitus, rheumatoid arthritis, inflammatory bowel disease, psoriasis, psoriatic arthritis, ankylosing spondylitis, systemic lupus erythematosus, Hashimoto's disease, graft-versus-host disease, Sjogren's syndrome, pernicious anemia, Addison disease, scleroderma, Goodpasture's syndrome, ulcerative colitis, autoimmune hemolytic anemia, sterility, myasthenia gravis, multiple sclerosis, Basedow's disease, thrombopenia purpura, Guillain-Barre syndrome, allergy, asthma, atopic disease, arteriosclerosis, myocarditis, cardiomyopathy, glomerular nephritis, hypoplastic anemia, and rejection after organ transplantation.

The term "immunomodulatory" refers to the modification, amplification, inhibition or reduction of one or more biological activities of the immune system which includes, but is not limited to, downregulation of immune response, augmentation of immune responses and changes of the inflammatory states mediated by changes in cytokine profile, cytotoxic activity and antibody production and their effects on immune and immune related cells.

The term "inflammatory disorders" refers to a condition in a subject characterized by inflammation, *e*.*g*., chronic inflammation. Illustrative, non-limiting examples of inflammatory disorders include, but are not limited to, Acute Optic Neuritis, Diabetic Neuropathy, Krabbe Disease, Acute Lung Injury, Crohn's Disease Celiac Disease, rheumatoid arthritis (RA), Inflammatory Bowel Disease (IBD), asthma, encephalitis, chronic obstructive pulmonary disease (COPD), inflammatory osteolysis, allergic disorders, septic shock, pulmonary fibrosis (e.g., idiopathic pulmonary fibrosis), inflammatory vacultides (*e*.*g*., polyarteritis nodosa, Wegner's granulomatosis, Takayasu's arteritis, temporal arteritis, and lymphomatoid granulomatosus), post-traumatic vascular angioplasty (*e.g*., restenosis after angioplasty), undifferentiated spondyloarthropathy, undifferentiated arthropathy, arthritis, inflammatory osteolysis, chronic hepatitis, and chronic inflammation resulting from chronic viral or bacterial infections.

The term "viral condition" refers to any disease caused by a virus. Suitable diseases include but are not limited to: Influenza, Adenovirus Infections, Respiratory Syncytial Disease, Rhinovirus Infections, Herpes Simplex, Chicken pox (Varicella), Measles (Rubeola), German Measles ( Rubella), Mumps (Epidemic Parotitis), Small Pox (Variola), Kawasaki Disease, Yellow Fever, Dengue Fever, Hepatitis A, Hepatitis B, NANB Hepatitis, Viral Gastroenteritis, Viral Fevers, Cytomegalovirus Disease, AIDS (HIV), Rabies, Polio, Ebola virus, Hemorrhagic Fever, Epstein-Barr, and disease including cancers that are caused by the virus that causes any of the preceding diseases.

The term "bacterial infection" refers to any infection caused by a medically relevant bacteria including but not limited to pertussis, leprosy, tuberculosis, toxic shock syndrome, food poisoning, salmonella, E. coli poisoning, Staphylococcus aureus, Clostridium difficile, sepsis, Lyme disease, cholera, dysentery, and others.

"Isolated cell population" means a cell population, isolated from the human or animal body, which is substantially free of one or more other cell populations that are normally associated with the cell population *in vivo* or *in vitro.*

The term "ligand inducer" means an agent or agents that result in increased production of such a ligand. A ligand inducer for a Toll-like receptor (TLR) ligand will yield increased TLR ligand, and is therefore essentially equivalent to the TLR ligand itself.

The term "MHC" (major histocompatibility complex) refers to a subset of genes that encode cell-surface antigen-presenting proteins. In humans, these genes are referred to as human leukocyte antigen (HLA) genes. The abbreviations MHC or HLA are used interchangeably.

The term "population of cells" means any number of cells greater than 1, but is at least 1 × 10³ cells, at least 1 × 10⁴ cells, at least 1 × 10⁵ cells, at least 1 × 10⁶ 6 cells, at least 1 × 10⁷ cells, at least 1 × 10⁸ cells, or at least 1 × 10⁹ or more cells. A population of cells also refers to cells in batch formation grown in bioreactors or other industrial methods intended to culture large amount of cells

In preferred embodiments of this invention, at least 50%, at least 55%, at least 60%, at least 65%, at least 70%, at least 75%, at least 80%, at least 85%, at least 90%, at least 91%, at least 92%, at least 93%, at least 94% or at least 95%, of the stem cells (% by cell number) in an initial cell population will be undifferentiated MSCs.

The term "significant expression" or its equivalent terms "positive" and "+" when used regarding a cell surface marker means that, in a cell population, more than 20%, preferably more than 30%, 40%, 50%, 60%, 70%, 80%, 90% 95%, 98%, 99%, or even 100% of the cells express the cell surface marker.

Expression of cell surface markers may be determined, for example, by means of flow cytometry for a specific cell surface marker using conventional methods and apparatus (for example a BECKMAN COULTER EPICS XL FACS system used with commercially available antibodies and standard protocols known in the art) that show a signal for a specific cell surface marker in flow cytometry above the background signal using conventional methods and apparatus. The background signal is defined as the signal intensity given by a non-specific antibody of the same isotype as the specific antibody used to detect each surface marker in conventional FACS analysis. For a marker to be considered positive the specific signal observed is stronger than 20%, preferably stronger than, 30%, 40%, 50%, 60%, 70%, 80%, 90%, 500%, 1000%, 5000%, 10000% or above, than the background signal intensity using conventional methods and apparatus. Furthermore, commercially available and known monoclonal antibodies against said cell-surface markers (e.g., cellular receptors and transmembrane proteins) can be used to identify relevant cells.

Expression of mRNA is determined by any suitable technology including, but not limited to, gene expression arrays (gene chips), mRNA-SEQ, northern blot, or the polymerase chain reaction (PCR), including quantitative PCR (qPCR) methods which include the use of reverse transcriptase. QPCR methods include, but are not limited to: probe based quantification, e.g. TaqMan^{®}; dye based quantification, e.g. SYBR green; digital PCR. The qPCR method can be one using an absolute quantification method, or a relative quantification method requiring normalization to housekeeping genes such as actin, GAPDH, or ribosomal subunits.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

**FIG. 1** shows gene expression data generated using a PCR array from MSC polarized to possess anti-inflammatory characteristics using the TLR3 ligand poly(I:C). Grey indicates at least two-fold induction of gene expression; black indicates a two-fold reduction in gene expression; and thick boxes indicate genes that were upregulated more than two-fold and selected for further validation (with the exception of PIAS2 which was reduced by at least two-fold) in FIG. 2.
**FIG. 2** shows validation of selected genes from the experiment in FIG. 1 using qPCR (**A** and **B**). Error bars indicate SEM.
**FIG. 3** shows induction of IL-6 and IL-8 secretion in MSC cells after treatment with TLR4 ligand (MSC1), or TLR4 ligand plus EPO and cobalt chloride (MSC1^{∗}).
**FIG. 4** shows induction of CCL5 and CXCL10 secretion in MSC cells after treatment with TLR3 ligand (MSC2), or TLR3 ligand plus EPO and cobalt chloride (MSC2*).
**FIG. 5** shows induction of an MSC1 phenotype, by expression of *TNFSF10 (TRAIL)* in MSC cells from different human donors, after treatment with TLR4 ligand (no*), or TLR4 ligand plus EPO and cobalt chloride (with*).
**FIG. 6** shows induction of an MSC2 phenotype, by expression of *CXCL9* in MSC cells from different human donors, after treatment with TLR3 ligand (no*), or TLR3 ligand plus EPO and cobalt chloride (with*).
**FIG. 7** shows a time course of induction of *TNFSF10 (TRAIL)* expression in MSC cells from MSC after treatment with TLR4 ligand (MSC1), or TLR4 ligand plus EPO and cobalt chloride (MSC1^{∗}).
**FIG. 8** shows a time course of induction of *CXCL9* expression in MSC cells from MSC after treatment with TLR3 ligand (MSC2), or TLR3 ligand plus EPO and cobalt chloride (MSC2*).
**FIG. 9** shows a transwell migration assay of MSC that are unstimulated (neg control) compared to MSC that have been stimulated with TLR4 ligand (MSC1), TLR4 ligand plus EPO and cobalt chloride (MSC1^{∗}), TLR3 ligand (MSC2), and TLR3 ligand plus EPO and cobalt chloride MSC2^{∗}). Error bares indicate SEM.
**FIG. 10** shows a cell proliferation/viability assay of MSC that are unstimulated (neg control) compared to MSC that have been stimulated with TLR4 ligand (MSC1), TLR4 ligand plus EPO and cobalt chloride(MSC1^{∗}), TLR3 ligand (MSC2), and TLR3 ligand plus EPO and cobalt chloride (MSC2*). Error bars indicate SEM.
**FIG. 11** Shows validation of a qPCR assay to measure *TNFSF 10 (TRAIL)* expression: shows an agarose gel of *TNFSF10 (TRAIL)* primer PCR amplification products.
FIG. 12 (A) shows a time course of *CXCL9* expression in MSC polarized to MSC2; (**B**) shows an agarose gel of CXCL9 primer PCR amplification products.

### DETAILED DESCRIPTION OF THE INVENTION

Practice of the invention employs - except for the invention itself - conventional techniques of cell culture, molecular biology, and microbiology, which are within the skill of those working in the art.

The invention provides an inducing, activating, polarizing or priming culture induction medium for a population of mesenchymal stem cells, comprising a Toll-like receptor (TLR) ligand or TLR-ligand inducer in combination with erythropoietin (EPO) and with exposure to hypoxia or hypoxia mimetic, plus additional, standard components of cell-culture medium known in the art and described here.

The invention also provides a culture-medium induction supplement comprising a Toll-like receptor (TLR) ligand or TLR-ligand inducer in combination with erythropoietin (EPO) and with exposure to hypoxia or hypoxia mimetic, which can be added to other, existing culture medium. Such a supplement might be appropriate where unusual components or concentrations of other components are appropriate for certain circumstances.

The invention also provides a hermetically-sealed culture vessel containing the culture induction medium or culture-medium induction supplement of the invention.

The invention also provides a method for preparing a culture induction medium as disclosed herein, comprising the steps of: (a) obtaining a culture medium; and (b) adding a Toll-like receptor (TLR) ligand or TLR-ligand inducer in combination with erythropoietin (EPO) and with exposure to hypoxia or hypoxia mimetic to the culture medium.

The invention also provides a composition comprising: (a) a culture medium according to the invention; and (b) stem cells.

The invention also provides a composition containing: (a) a culture medium according to the invention; and (b) a solid surface. In certain embodiments, a solid surface is any tissue culture compatible surface including tissue culture plates, flasks and bottles of any size for use in 2D cell culture. In certain embodiments, a solid surface is a microcarrier or any other support matrix for cells used in 3D culture.

The invention also provides the use of a culture medium of the invention for inducing, activating or priming a population of mesenchymal stem cells.

The invention also provides an ex-vivo method for inducing, activating or priming a population of mesenchymal stem cells, comprising: (a) providing a population of mesenchymal stem cells; (b) providing a culture medium of the invention; (c) contacting the stem cells with the culture medium; and (d) culturing the cells under appropriate conditions.

In one aspect the invention provides the use of a Toll-like receptor (TLR) ligand or TLR-ligand inducer in combination with erythropoietin and with exposure to hypoxia or hypoxia mimetic in the manufacture of a cellular therapy medicament. Accordingly in one embodiment the invention also provides a method of manufacture of a cellular therapy medicament, comprising: (a) providing a population of mesenchymal stem cells; (b) providing a culture medium of the invention; (c) contacting the stem cells with the culture medium; and (d) culturing the cells under appropriate conditions. The invention also provides the use of a composition comprising : (a) a culture medium according to the invention; and (b) stem cells, for manufacturing a cellular therapy medicament. The invention also provides the use of a composition comprising : (a) a culture medium according to the invention; and (b) a solid surface, for manufacturing a cellular therapy medicament.

Said medicaments are of use in the treatment, repair, prophylaxis, and/or amelioration of damaged tissues, or one or more symptoms associated with inflammatory and/or immune disorders such as but not limited to autoimmune diseases, inflammatory disorders, and immunologically mediated diseases including rejection of transplanted organs and tissues and cancer. A cellular therapy medicament of the invention comprises a prophylactically or therapeutically effective amount of stem cells and a pharmaceutical carrier. Particularly preferred are stem cells of mesenchymal origin. Examples of dosages and dosage regimens for each of these cell types are known in the art. Suitable pharmaceutical carriers are known in the art and are preferably those approved by a regulatory agency of the US Federal or a state government or listed in the U S Pharmacopeia, or European Pharmacopeia, or other generally recognized pharmacopeia for use in animals, and more particularly in humans. The term "carrier" refers to a diluent, adjuvant, excipient, or vehicle with which the therapeutic agent is administered. The composition, if desired, can also contain minor amounts of pH buffering agents. Examples of suitable pharmaceutical carriers are described in "Remington's Pharmaceutical Sciences" by E W Martin. Such compositions will contain a prophylactically or therapeutically effective amount of a prophylactic or therapeutic agent preferably in purified form, together with a suitable amount of carrier so as to provide the form for proper administration to the subject. The formulation should suit the mode of administration. In a preferred embodiment, the medicaments are sterile and in suitable form for administration to a subject, preferably an animal subject, more preferably a mammalian subject, and most preferably a human subject.

In certain embodiments, the methods, cells and induction medium of this disclosure are for the treatment of acute pain or chronic pain. In certain embodiments, the pain is not associated with a specific diagnosis. In certain embodiments, the pain is associated with a trauma. In certain embodiments, the pain is back pain. In certain embodiments, the pain is associated with a herniated spinal disc or degenerative disc disease. In certain embodiments, the pain is neuropathic. In certain embodiments, the pain is due to sciatica.

In certain embodiments, the methods, cells and induction medium of this disclosure are for the treatment of cancer. In certain embodiments, the methods, cells and induction medium of this disclosure are for the treatment of tumors. In certain embodiments, the methods, cells and induction medium of this disclosure are for augmenting the treatment of cancer. In certain embodiments, the cancer is Acute Lymphoblastic Leukemia, Adult; Acute Lymphoblastic Leukemia, Childhood; Acute Myeloid Leukemia, Adult; Acute Myeloid Leukemia, Childhood; Adreno cortical Carcinoma; AIDS-Related Cancers; AIDS-Related Lymphoma; Anal Cancer; Appendix Cancer; Astrocytomas; Atypical Teratoid/Rhabdoid Tumor; Basal Cell Carcinoma; Bile Duct Cancer, Extrahepatic; Bladder Cancer; Bone Cancer, Osteosarcoma and Malignant Fibrous Histiocytoma; Brain Stem Glioma; Brain Tumor; Central Nervous System Embryonal Tumors; Astrocytomas; Craniopharyngioma; Ependymoblastoma; Brain Tumor, Ependymoma; Medulloblastoma; Medulloepithelioma; Pineal Parenchymal Tumors of Intermediate Differentiation; Supratentorial Primitive Neuro ectodermal Tumors and Pineoblastoma; Brain and Spinal Cord Tumors; Breast Cancer; Breast Cancer, Male; Bronchial Tumors; Burkitt Lymphoma; Carcinoid Tumor; Central Nervous System Atypical Teratoid/Rhabdoid Tumor; Central Nervous System Embryonal Tumors; Central Nervous System (CNS) Lymphoma, Cervical Cancer; Primary; Cervical Cancer; Childhood Cancers; Chordoma; Chronic Lymphocytic Leukemia; Chronic Myelogenous Leukemia; Chronic Myeloproliferative Disorders; Colon Cancer; Colorectal Cancer; Craniopharyngioma; Cutaneous T-Cell Lymphoma; Embryonal Tumors, Central Nervous System; Endometrial Cancer; Ependymoblastoma; Ependymoma; Esophageal Cancer; Esthesioneuroblastoma; Ewing Sarcoma Family of Tumors; Extracranial Germ Cell Tumor; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastrointestinal Carcinoid Tumor; Gastrointestinal Stromal Tumor (GIST); Germ Cell Tumor, Extracranial; Germ Cell Tumor, Extragonadal; Germ Cell Tumor, Ovarian; Gestational Trophoblastic Tumor; Glioma; Hairy Cell Leukemia; Head and Neck Cancer; Heart Cancer; Hepatocellular (Liver) Cancer, Adult (Primary); Hepatocellular (Liver) Cancer; Histiocytosis, Langerhans Cell; Hodgkin Lymphoma, Adult; Hodgkin Lymphoma, Childhood; Hypopharyngeal Cancer; Intraocular Melanoma; Islet Cell Tumors (Endocrine Pancreas); Kaposi Sarcoma; Kidney (Renal Cell) Cancer; Kidney Cancer; Langerhans Cell Histiocytosis; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer; Lung Cancer, Non-Small Cell; Lung Cancer, Small Cell; Lymphoma, AIDS-Related; Lymphoma, Burkitt; Lymphoma, Cutaneous T-Cell; Lymphoma, Hodgkin, Adult; Lymphoma, Hodgkin, Childhood; Lymphoma, Non-Hodgkin, Adult; Lymphoma, Non-Hodgkin, Childhood; Lymphoma, Primary Central Nervous System (CNS); Macroglobulinemia, Waldenström; Malignant Fibrous Histiocytoma of Bone and Osteosarcoma; Medulloblastoma; Medulloepithelioma; Melanoma; Melanoma, Intraocular (Eye); Merkel Cell Carcinoma; Mesothelioma, Adult Malignant; Mesothelioma; Metastatic Squamous Neck Cancer with Occult Primary; Mouth Cancer; Multiple Endocrine Neoplasia Syndrome; Multiple Myeloma/Plasma Cell Neoplasm; Mycosis Fungoides; Myelodysplastic Syndromes; Myelodysplastic/Myeloproliferative Neoplasms; Myelogenous Leukemia, Chronic; Myeloid Leukemia, Adult Acute; Myeloid Leukemia, Childhood Acute; Myeloma, Multiple; Myeloproliferative Disorders, Chronic; Nasal Cavity and Paranasal Sinus Cancer; Nasopharyngeal Cancer;; Neuroblastoma; Non-Hodgkin Lymphoma, Adult; Non-Hodgkin Lymphoma, Childhood; Non-Small Cell Lung Cancer; Oral Cancer; Oral Cavity Cancer, Lip and; Oropharyngeal Cancer; Osteosarcoma and Malignant Fibrous Histiocytoma of Bone; Ovarian Cancer; Ovarian Epithelial Cancer; Ovarian Germ Cell Tumor; Ovarian Low Malignant Potential Tumor; Pancreatic Cancer; Pancreatic Cancer, Islet Cell Tumors; Papillomatosis; Paranasal Sinus and Nasal Cavity Cancer; Parathyroid Cancer; Penile Cancer; Pharyngeal Cancer; Pineal Parenchymal Tumors of Intermediate Differentiation; Pituitary Tumor; Plasma Cell Neoplasm/Multiple Myeloma; Pleuropulmonary Blastoma; Pregnancy and Breast Cancer; Primary Central Nervous System (CNS) Lymphoma; Prostate Cancer; Rectal Cancer; Renal Cell (Kidney) Cancer; Renal Pelvis and Ureter, Transitional Cell Cancer; Respiratory Tract Cancer with Chromosome 15 Changes; Retinoblastoma; Rhabdomyosarcoma; Salivary Gland Cancer; Salivary Gland Cancer; Sarcoma, Ewing Sarcoma Family of Tumors; Sarcoma, Kaposi; Sarcoma, Soft Tissue, Adult; Sarcoma, Soft Tissue, Childhood; Sarcoma, Uterine; Sézary Syndrome; Skin Cancer (Nonmelanoma); Skin Cancer; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Cell Carcinoma; Squamous Neck Cancer with Occult Primary, Metastatic; Stomach (Gastric) Cancer; Supratentorial Primitive Neuroectodermal Tumors; T-Cell Lymphoma, Cutaneous; Testicular Cancer; Throat Cancer; Thymoma and Thymic Carcinoma; Thyroid Cancer; Transitional Cell Cancer of the Renal Pelvis and Ureter; Trophoblastic Tumor, Gestational; Unknown Primary Site, Carcinoma of; Ureter and Renal Pelvis, Transitional Cell Cancer; Urethral Cancer; Uterine Cancer, Endometrial; Uterine Sarcoma; Uveal melanoma; Vaginal Cancer; Vulvar Cancer; Waldenström Macroglobulinemia or Wilms Tumor.

In certain embodiments, the methods, cells and induction medium of this disclosure are for administration to a subject in need of treatment for a cancer, autoimmune disorder or inflammatory disorder. In certain, embodiments, the methods cells and induction medium of this disclosure encompass different routes of administration. In certain embodiments, the routes of administration are subcutaneous, intraparietal, intramuscular, intravenous, intratumor, intraocular, intraretinal, intravitreal or intracranial.

In certain embodiments, the methods, cells and induction medium of this disclosure are for administration to a subject in need of treatment for a cancer, autoimmune disorder or immune mediated inflammatory disease. In certain, embodiments, the methods cells and induction medium of this disclosure encompass different dosage frequencies. In certain embodiments, cells and mendicants of this disclosure are administered once a day, once a week, once a month, or once a year. In certain embodiments, cells and methods of this disclosure are administered twice a day, twice a week, twice a month, or twice a year. In certain embodiments, cells and methods of this disclosure are administered thrice a day, thrice a week, thrice a month, or thrice a year. In certain embodiments, cells and methods of this disclosure are for administration four times a day, four times a week, four times a month, or four times a year. In certain embodiments, a primary treatment is followed by a maintenance dose 1, 2, 3, 4, 5, 6, 7, 8, 9,10, 11 or 12 times a year. In certain embodiments, the maintenance dose is continued for at least 1, 2, 3, 4, 5, 6, 7, 8, 9,10 or more years. In certain embodiments, at least 1×10⁶cells are administered per dose. In certain embodiments, at least 2×10⁶cells are administered per dose. In certain embodiments, at least 3×10⁶ cells are administered per dose. In certain embodiments, at least 4×10⁶ cells are administered per dose. In certain embodiments, at least 5×10⁶ cells are administered per dose. In certain embodiments, at least 6×10⁶ cells are administered per dose. In certain embodiments, cells are administered. In certain embodiments, at least 7×10⁶cells are administered per dose. In certain embodiments, at least 8×10⁶ cells are administered per dose. In certain embodiments, at least 9×10⁶ cells are administered per dose. In certain embodiments, at least 1×10⁷ cells are administered per dose. In certain embodiments, at least 2×10⁷ cells are administered per dose. In certain embodiments, at least 3×10⁷ cells are administered per dose. In certain embodiments, at least 4×10⁷ cells are administered per dose. In certain embodiments, at least 5×10⁷ cells are administered per dose. In certain embodiments, at least 6×10⁷ cells are administered per dose. In certain embodiments, at least 7×10⁷ cells are administered per dose. In certain embodiments, at least 8×10⁷ cells are administered per dose. In certain embodiments, at least 9×10⁷ cells are administered per dose. In certain embodiments, at least 1×10⁸ cells are administered per dose. In certain embodiments, at least 2×10⁸cells are administered per dose. In certain embodiments, at least 3×10⁸ cells are administered per dose. In certain embodiments, at least 4×10⁸ cells are administered per dose. In certain embodiments, at least 5×10⁸cells are administered per dose. In certain embodiments, at least 6×10⁸ cells are administered per dose. In certain embodiments, at least 7×10⁸cells are administered per dose. In certain embodiments, at least 8×10⁸ cells are administered per dose. In certain embodiments, at least 9×10⁸cells are administered per dose. In certain embodiments, at least 1×10⁹ cells are administered per dose. In certain embodiments, at least 2×10⁹cells are administered per dose. In certain embodiments, at least 3×10⁹cells are administered per dose.

The medicament of the invention may be in a variety of forms. These include, for example, semi-solid, and liquid dosage forms, such as lyophilized preparations, liquid solutions or suspensions, injectable and infusible solutions, etc., the medicament is preferably injectable.

In certain embodiments, medicaments are for treating or repairing damaged tissue (preferably mesenchymal tissue), and/or for the treatment, modulation, prophylaxis, and/or amelioration of one or more symptoms associated with inflammatory and/or immune disorders. Accordingly the methods and cells of the invention are of use in the treatment of any disorder characterized by either or all of said symptoms. A representative non-exhaustive list of such disorders is provided in the definitions section. Particularly preferred is a medicament for the treatment of immune-mediated inflammatory diseases. Further preferred is a medicament for the treatment of diabetes mellitus, rheumatoid arthritis (RA), inflammatory bowel disease (IBD, including Crohn's disease and/or Ulcerative Colitis) and multiple sclerosis (MS). The invention also provides the use of a Toll-like receptor (TLR) ligand or TLR-ligand inducer in combination with erythropoietin and with exposure to hypoxia or hypoxia mimetic for mesenchymal stem cell culture.

The specific ingredients and ratio of ingredients of the culture medium, supplements and compositions of the invention can vary according to particular needs and applications. Likewise, the precise steps of the methods of the invention can vary according to particular needs and applications. The culture medium, supplements, methods, compositions and uses according to this invention may be optimized by routine experimentation. For example, if a desired outcome is an anti-inflammatory therapeutic effect, if a culture medium, supplement or composition will specifically contain a TLR3 ligand or TLR-ligand inducer in combination with erythropoietin and with exposure to hypoxia or hypoxia mimetic (cobalt chloride or desferrioxamine) by contrast, if a desired outcome is a pro-immune therapeutic effect, if a culture medium, supplement or composition will specifically contain a TLR4 ligand or TLR-ligand inducer in combination with erythropoietin and with exposure to hypoxia or hypoxia mimetic. The amount of each of the ingredients described herein can be optimized independently of the other ingredients by routine optimization or one or more ingredients can be added or removed. A culture medium can be tested for its ability to support induction, activation or priming of mesenchymal stem cells by testing it alongside or in place of a known culture medium or method. The culture medium, supplements, methods, compositions and uses of the invention are described in more detail below.

The induction medium of the invention comprises a Toll-like receptor (TLR) ligand or TLR-ligand inducer in combination with erythropoietin and with exposure to hypoxia or hypoxia mimetic. In one aspect the induction medium of the invention comprises a Toll-like receptor (TLR) ligand or TLR-ligand inducer. In an alternative aspect the induction medium of the invention comprises erythropoietin and exposure to hypoxia or hypoxia mimetic. In a further aspect the induction medium of the invention comprises a Toll-like receptor (TLR) ligand or TLR-ligand inducer in combination with erythropoietin and with exposure to hypoxia or hypoxia mimetic. In certain embodiments, TLR ligand is a TLR4 ligand. In certain embodiments, the TLR ligand is a TLR3 ligand

The induction medium of the invention may comprise two or more, three or more, 4-, 5-,6-,7-,8-,9-, 10- or more, combinations of a Toll-like receptor (TLR) ligand or TLR-ligand inducer in combination with erythropoietin (EPO) and with exposure to hypoxia or hypoxia mimetic.

An induction medium of the invention may comprise between about 0.10 picomolar (pM) and about 100 millimolar (mM) of a TLR ligand or TLR-ligand inducer in combination with between about 0.5 mU/mL and about 100 mU/mL erythropoietin (EPO) and with exposure to about 0.5 to about 2% oxygen conditions (hypoxia) or hypoxia mimetic such as cobalt chloride or desferrioxamine, at a concentration of about 10 micromolar to about 1 mM, or any other combination of the above TLR ligand or TLR-ligand inducer, erythropoietin, and hypoxia.

The TLR3 ligand used in the induction culture medium may be IL4, IL13, poly(A:U), poly(I:C), and combinations thereof, and may be delivered by incubation, transfection, transduction, by carrier molecules, or by combinations thereof. Preferably, the TLR3 ligand or agonist is poly(I:C).

The TLR4 ligand used in the induction culture medium may be aminoalkyl glucosaminide 4-phosphates, interferons, TNF-alpha, GM-CSF, lipopolysaccharide (LPS), and combinations thereof, and may be delivered by incubation, transfection, transduction, by carrier molecules, or by combinations thereof. Preferably, the TLR4 ligand or agonist is LPS.

The TLR3 ligand or agonist may be provided in an amount from about 10 pg/mL to about 100 µg/mL, from about 100 pg/mL to about 100 µg/mL, from about 1 ng/mL to about 100 µg/mL, from about 5 ng/mL to about 100 µg/mL, from about 10 ng/mL to about 100 µg/mL, from about 100 ng/mL to about 100 µg/mL, from about 0.1 µg/mL to about 50 µg/mL, from about 0.1 µg/mL to about 10 µg/mL, from about 0.25 µg/mL to about 7.5 µg/mL, from about 0.5 µg/ mL to about 5 µg/mL, from about 1 µg/mL to about 2.5 µg/mL, and preferably from about 1 µg/mL to about 1.5 µg/mL in culture medium or supplement as noted above.

In certain embodiments, the TLR3 ligand is poly(I:C), and is provided in an amount from about 10 pg/mL to about 100 µg/mL, from about 100 pg/mL to about 100 µg/mL, from about 1 ng/mL to about 100 µg/mL, from about 5 ng/mL to about 100 µg/mL, from about 10 ng/mL to about 100 µg/mL, from about 100 ng/mL to about 100 µg/mL, from about 0.1 µg/mL to about 50 µg/mL, from about 0.1 µg/mL to about 10 µg/mL, from about 0.25 µg/mL to about 7.5 µg/mL, from about 0.5 µg/ mL to about 5 µg/mL, from about 1 µg/mL to about 5 µg/mL, and from about 1 µg/mL to about 2.5 µg/mL in. In certain embodiments, the poly(I:C) is provided in an amount of about 1 µg/mL. In certain embodiments, the poly(I:C) is provided in an amount of about 2µg/mL. In certain embodiments, the poly(I:C) is provided in an amount of about 3 µg/mL. In certain embodiments, the poly(I:C) is provided in an amount of about 4 µg/mL. In certain embodiments, the poly(I:C) is provided in an amount of about 5 µg/mL. In certain embodiments, the poly(I:C) is provided in an amount of about 6 µg/mL. In certain embodiments, the poly(I:C) is provided in an amount of about 7 µg/mL. In certain embodiments, the poly(I:C) is provided in an amount of about 8 µg/mL. In certain embodiments, the poly(I:C) is provided in an amount of about 9 µg/mL. In certain embodiments, the poly(I:C) is provided in an amount of about 10 µg/mL. In certain embodiments, the poly(I:C) is provided in an amount of less than about 100 ng/mL. In certain embodiments, the poly(I:C) is provided in an amount of less than about 50 ng/mL. In certain embodiments, the poly(I:C) is provided in an amount of less than about 20 ng/mL. In certain embodiments, the poly(I:C) is provided in an amount of less than about 10 ng/mL. In certain embodiments, the poly(I:C) is provided in an amount of less than about 50 ng/mL.

The TLR4 ligand or agonist may be provided in an amount from about 10 pg/mL to about 10 µg/mL, from about 100 pg/mL to about 10 µg/mL, from about 1 ng/mL to about 1 µg/mL, from about 5 ng/mL to about 1 µg/mL, from about 10 ng/mL to about 1 µg/mL, from about 100 ng/mL to about 1 µg/mL, preferably from about 5 ng/mL to about 50 ng/mL, and also preferably from about 5 ng/mL to about 25 ng/mL in culture medium or supplement as noted above.

In certain embodiments, the TLR4 ligand is LPS. In certain embodiments, the LPS is present in an amount from about 10 pg/mL to about 10 µg/mL, from about 100 pg/mL to about 10 µg/mL, from about 1 ng/mL to about 1 µg/mL, from about 5 ng/mL to about 1 µg/mL, from about 10 ng/mL to about 1 µg/mL, from about 100 ng/mL to about 1 µg/mL, preferably from about 5 ng/mL to about 50 ng/mL, and also preferably from about 5 ng/mL to about 25 ng/mL. In certain embodiments, the LPS is present at a concentration of about 5 ng/mL. In certain embodiments, the LPS is present at a concentration of about 10 ng/mL. In certain embodiments, the LPS is present at a concentration of about 15 ng/mL. In certain embodiments, the LPS is present at a concentration of about 20 ng/mL. In certain embodiments, the LPS is present at a concentration of about 25 ng/mL. In certain embodiments, the LPS is present at a concentration of about 30 ng/mL. In certain embodiments, the LPS is present at a concentration of about 35 ng/mL. In certain embodiments, the LPS is present at a concentration of about 40 ng/mL. In certain embodiments, the LPS is present at a concentration of about 45 ng/mL. In certain embodiments, the LPS is present at a concentration of about 50 ng/mL. In certain embodiments, the LPS is present at a concentration of less than about 100 ng/mL. In certain embodiments, the LPS is present at a concentration of less than about 50 ng/mL. In certain embodiments, the LPS is present at a concentration of less than about 20 ng/mL. In certain embodiments, the LPS is present at a concentration of less than about 10 ng/mL.

In certain embodiments, the induction culture medium of the invention comprises an incubation in a hypoxic or oxygen depleted environment. In certain embodiments, a hypoxic environment possess less than 2% oxygen. In certain embodiments, a hypoxic environment possess less than 1.5% oxygen. In certain embodiments, a hypoxic environment possess less than 1.0% oxygen. In certain embodiments, a hypoxic environment possess less than 0.5% oxygen. In certain embodiments, a hypoxic environment possess effectively 0% oxygen. In certain embodiments, a hypoxic environment possess between 0.5% and 2.0% oxygen. In certain embodiments, a hypoxic environment possess between 0.5% and 1.5% oxygen. In certain embodiments, a hypoxic environment possess between 0.5% and 1.0% oxygen. In certain embodiments, a hypoxic environment possess between 1.0% and 2.0% oxygen. In certain embodiments, a hypoxic environment possess between 1.5% and 2.0% oxygen.

In certain embodiments, the induction culture medium of the invention comprises cobalt chloride. In certain embodiments, cobalt chloride is present at a concentration of about 50 µM. In certain embodiments, cobalt chloride is present at a concentration of about 100 µM. In certain embodiments, cobalt chloride is present at a concentration of about 200 µM. In certain embodiments, cobalt chloride is present at a concentration of about 300 µM. In certain embodiments, cobalt chloride is present at a concentration of about 400 µM. In certain embodiments, cobalt chloride is present at a concentration of about 500 µM. In certain embodiments, cobalt chloride is present at a concentration of about 600 µM. In certain embodiments, cobalt chloride is present at a concentration of about 700µM. In certain embodiments, cobalt chloride is present at a concentration of about 800µM. In certain embodiments, cobalt chloride is present at a concentration of about 900µM. In certain embodiments, cobalt chloride is present at a concentration of about 1 mM. In certain embodiments, cobalt chloride is present at a concentration of from about 10 µM to about 1 mM. In certain embodiments, cobalt chloride is present at a concentration of from about 10 µM about 800 µM. In certain embodiments, cobalt chloride is present at a concentration of from about 10 µM to about 500 µM. In certain embodiments, cobalt chloride is present at a concentration of from about 10 µM to about 400 µM. In certain embodiments, cobalt chloride is present at a concentration of from about 10 µM to about 300 µM. In certain embodiments, cobalt chloride is present at a concentration of from about 50 µM to about 300 µM. In certain embodiments, cobalt chloride is present at a concentration of from about 100 µM to about 300 µM. In certain embodiments, cobalt chloride is present at a concentration of from about 150 µM to about 300 µM.

In certain embodiments, the induction culture medium of the invention comprises desferrioxamine. In certain embodiments, desferrioxamine is present at a concentration of about 50 µM. In certain embodiments, desferrioxamine is present at a concentration of about 200 µM. In certain embodiments, desferrioxamine is present at a concentration of about 300 µM. In certain embodiments, desferrioxamine is present at a concentration of about 400 µM. In certain embodiments, desferrioxamine is present at a concentration of about 500 µM. In certain embodiments, desferrioxamine is present at a concentration of about 600 µM. In certain embodiments, desferrioxamine is present at a concentration of about 700µM. In certain embodiments, desferrioxamine is present at a concentration of about 800µM. In certain embodiments, desferrioxamine is present at a concentration of about 900µM. In certain embodiments, desferrioxamine is present at a concentration of about 1 mM. In certain embodiments, desferrioxamine is present at a concentration of from about 10 µM to about 1 mM. In certain embodiments, desferrioxamine is present at a concentration of from about 10 µM about 800 µM. In certain embodiments, desferrioxamine is present at a concentration of from about 10 µM to about 500 µM. In certain embodiments, desferrioxamine is present at a concentration of from about 10 µM to about 400 µM. In certain embodiments, desferrioxamine is present at a concentration of from about 10 µM to about 300 µM. In certain embodiments, desferrioxamine is present at a concentration of from about 50 µM to about 300 µM. In certain embodiments, desferrioxamine is present at a concentration of from about 100 µM to about 300 µM. In certain embodiments, desferrioxamine is present at a concentration of from about 150 µM to about 300 µM.

In certain embodiments, the induction culture medium of the invention comprises erythropoietin. In certain embodiments, the induction culture medium of the invention comprises recombinant erythropoietin. In certain embodiments, the induction culture medium of the invention comprises human recombinant erythropoietin. In certain embodiments, the amount of erythropoietin is between about 0.1 ng/mL and about 1.0 mg/mL. In certain embodiments, the amount of erythropoietin is between about 0.1 ng/mL and about 100 ng/mL. In certain embodiments, the amount of erythropoietin is between about 0.1 ng/mL and about 50 ng/mL. In certain embodiments, the amount of erythropoietin is between about 0.1 ng/mL and about 10 ng/mL. In certain embodiments, the amount of erythropoietin is between about 0.1 ng/mL and about 1.0 ng/mL. In certain embodiments, the amount of erythropoietin is between about 0.2 ng/mL and about 0.8 ng/mL. In certain embodiments, the amount of erythropoietin is between about 0.3 ng/mL and about 0.6 ng/mL. In certain embodiments, the amount of erythropoietin is less than 10 mg/mL. In certain embodiments, the amount of erythropoietin is less than 5 mg/mL. In certain embodiments, the amount of erythropoietin is less than 1 mg/mL. In certain embodiments, the amount of erythropoietin is less than 100 ng/mL. In certain embodiments, the amount of erythropoietin is less than 30ng/mL. In certain embodiments, the amount of erythropoietin is less than 10 ng/mL. In certain embodiments, the amount of erythropoietin is less than 5 ng/mL. In certain embodiments, the amount of erythropoietin is less than 4 ng/mL. In certain embodiments, the amount of erythropoietin is less than 1 ng/mL. In certain embodiments, the amount of erythropoietin is less than 0.8 ng/mL. In certain embodiments, the amount of erythropoietin is less than 1 ng/mL. In certain embodiments, the amount of erythropoietin is less than 5U/mL. In certain embodiments, the amount of erythropoietin is less than 1U/mL. In certain embodiments, the amount of erythropoietin is less than 0.5 U/mL. In certain embodiments, the amount of erythropoietin is less than 0.1U/mL. In certain embodiments, the amount of erythropoietin is less than 0.05 U/mL.

Cell induction medium typically contain a large number of ingredients, which are necessary to support maintenance of the cultured cells. An induction medium of the invention will therefore normally contain many other ingredients in addition to a Toll-like receptor (TLR) ligand or TLR-ligand inducer in combination with erythropoietin and with exposure to hypoxia or hypoxia mimetic (cobalt chloride or desferrioxamine). Suitable combinations of ingredients can readily be formulated by the skilled person, taking into account the following disclosure. An induction medium according to the invention will generally be a nutrient solution comprising standard cell culture ingredients, such as amino acids, vitamins, trace metals, inorganic salts, a carbon energy source, and a buffer, as described in more detail below.

An induction medium of the invention may contain serum. Serum contains cellular and non-cellular factors and components that may be necessary for viability and expansion. Serum obtained from any appropriate source may be used, including fetal bovine serum (FBS), bovine serum (BS), calf serum (CS), fetal calf serum (FCS), newborn calf serum (NCS), goat serum (GS), horse serum (HS), porcine serum, sheep serum, rabbit serum, rat serum (RS), etc. It is also within the scope of the invention that if said MSC are of human origin, the cell induction medium is supplemented with a human serum, preferably of autologous origin. It is understood that sera can be heat inactivated at 55-65 deg. C if deemed necessary to inactivate components of the complement cascade. Where a serum replacement is used, it may be used at between about 2% and about 40% by volume of the medium, according to conventional techniques.

In other embodiments, an induction medium of the invention may contain a serum replacement. Various different serum replacement formulations are commercially available and are known to the skilled person, such as but not limited to serum albumin, serum transferrin, selenium, and recombinant proteins including but not limited to insulin, platelet-derived growth factor (PDGF), and basic fibroblast growth factor (bFGF). Where a serum replacement is used, it may be used at between about 2% and about 40% by volume of the medium, according to conventional techniques. In other embodiments, an induction medium of the invention may be serum-free and/or serum replacement-free. A serum-free medium is one that contains no animal serum of any type. Serum-free medium may be preferred to avoid possible xeno-contamination of the stem cells. A serum replacement-free medium is one that has not been supplemented with any commercial serum replacement formulation.

An induction medium of the invention will normally be formulated in deionized, distilled water. An induction medium of the invention will typically be sterilized prior to use to prevent contamination, e.g. by ultraviolet light, heating, irradiation or filtration. The induction medium may be frozen (e.g. at -20°C or -80°C) for storage or transport. Antimicrobial agents are also typically used in medium to mitigate bacterial, mycoplasmal, and fungal contamination. The medium may contain one or more antimicrobial agents or antibiotics to prevent contamination. Typically, antibiotics or anti-mycotic compounds used are mixtures of penicillin/streptomycin, but can also include, but are not limited to amphotericin (Fungizone^{®}), ampicilhn, gentamicin, bleomycin, hygromacin, kanamycin, mitomycin, *etc.*

In one embodiment of the invention, the culture medium is a medium that has been conditioned by the addition of cells induced by a Toll-like receptor (TLR) ligand or TLR-ligand inducer in combination with erythropoietin and with exposure to hypoxia or hypoxia mimetic (cobalt chloride or desferrioxamine). Conditioned medium is produced by culturing a population of said cells in an induction medium for a time sufficient to condition the medium, then harvesting the conditioned medium. Where a conditioned medium is used, the medium may be conditioned on mammalian cells, e.g. mouse cells or human cells. Various different types of mammalian cells may be used to produce conditioned medium suitable for mesenchymal stem cell induction.

An induction medium may be a 1x formulation or a concentrated formulation, e.g. a 2x to 250x concentrated medium formulation. In a 1x formulation each ingredient in the medium is at the concentration intended for cell induction. In a concentrated formulation one or more of the ingredients is present at a higher concentration than intended for cell induction. Induction medium can be concentrated using known methods e.g. salt precipitation or selective filtration. A concentrated medium may be diluted for use with water (preferably deionized and distilled) or any appropriate solution, e.g. an aqueous saline solution, an aqueous buffer or a culture medium.

An induction medium as disclosed herein may be capable of inducing, activating or priming a population of stem cells in a multipotent, undifferentiated and proliferative state for only a single passage or population doubling under appropriate conditions. Stem cells are considered to be in a multipotent, undifferentiated and proliferative state if they exhibit certain characteristics as described in more detail elsewhere herein. Appropriate conditions can be selected by the skilled person from those normally used for mesenchymal stem cell culture.

As noted elsewhere herein, the invention also provides a hermetically-sealed vessel containing an induction medium of the invention. Hermetically-sealed vessels may be preferred for transport or storage of the induction medium, to prevent contamination. The vessel may be any suitable vessel, such as a bioreactor, a flask, a plate, a bottle, a jar, a vial or a bag. As noted elsewhere herein, the invention also provides a method for preparing an induction medium, comprising the steps of: (a) obtaining a culture medium; and (b) adding a Toll-like receptor (TLR) ligand or TLR-ligand inducer in combination with erythropoietin (EPO) and with exposure to hypoxia or hypoxia mimetic (cobalt chloride or desferrioxamine) to the culture medium. Various different methods for preparing induction medium are envisaged, depending on the specific ingredients to be included in the induction medium. For example, a method for preparing an induction medium may comprise the steps of: (a) obtaining a culture medium; and (b) adding a TLR ligand or TLR-ligand inducer in combination with erythropoietin (EPO) and with exposure to hypoxia or hypoxia mimetic (cobalt chloride or desferrioxamine) to the culture medium. In one embodiment, a method for preparing an induction medium may comprise the steps of: (a) obtaining a culture medium; and (b) adding a TLR ligand, EPO and cobalt chloride to the culture medium.

The induction medium of the invention can be used to induce, activate or prime a population of mesenchymal stem cells. Accordingly, the invention provides the use of any induction medium as disclosed herein for inducing, activating or priming a population of mesenchymal stem cells into discrete uniform phenotypes for cell-based therapy.

In certain embodiments, the induction medium disclosed herein induces or reduces expression of certain genes which can be measured by methods known to one skilled in the art including, but not limited to: PCR; qPCR; qRT-PCR; semi quantitative RT-PCR; digital PCR; northern blot; mRNA-SEQ; microarrays; and others. In certain embodiments, the induction medium disclosed herein increases or decreases protein levels which can be measured by methods known to one skilled in the art including, but not limited to: antibody based assays; enzyme-linked immunosorbent assay (ELISA); immuno or western blot; flow cytometry, mass spectrometry and others. In certain embodiments, the induction medium disclosed herein induces activation or attenuation of cell signaling pathways which can be measured by method known to skilled in the art including, but no limited to: kinase assay; protein phosphorylation/dephosphorylation measurements; protein ubiquitination/deubiquitination measurements; protein acetylation/deacetylation measurements; protein degradation/stability measurements; measurements of second messengers such as calcium or diacylglycerol; or monitoring of cleavage from an inactive to active form.

In certain embodiments, the induction medium disclosed herein leads to measurable changes in the gene expression, protein levels, or cell signaling pathways, of a cell population. In certain embodiments, the change is an increase in gene expression, protein levels or cell signaling. In certain embodiments, the change is any statistically significant change as measured between an unstimulated or control sample, and a stimulated or test sample. In certain embodiments, the change between an unstimulated or control sample, and a stimulated or test sample, is an increase of at least 2-fold, 3-fold, 4-fold, 5-fold, 6-fold, 7-fold, 8-fold, 9-fold, 10-fold or more. In certain embodiments, the change between an unstimulated or control sample, and a stimulated or test sample, is an increase of at least 100-fold or more. In certain embodiments, the change between an unstimulated or control sample, and a stimulated or test sample, is a decrease of at least 2-fold, 3-fold, 4-fold, 5-fold, 6-fold, 7-fold, 8-fold, 9-fold, 10-fold or more. In certain embodiments, the change between an unstimulated or control sample, and a stimulated or test sample, is a decrease of at least 100-fold or more.

In certain embodiments, the induction medium which comprises a TLR3 ligand induces mRNA expression of any of the following genes by at least 2-fold when compared to an unstimulated cell population: CXCL9; EGFR; IRF1; A2M; FAS; IL2RG; MMP3; GBP1; ISG15; FCGR1; NFKB1; NOS2A; USF1; YY1; JAK2; STA2, STAT4; STATS; SOCS1; or IRF1. In certain embodiments, the induction medium which comprises a TLR3 ligand reduces mRNA expression of any of the following genes by at least 2-fold when compared to an unstimulated cell population: EPOR; F2R; STAM; PDGFRA; PIAS2; MYC; SH2B1; or CSF2RB. In certain embodiments, the induction medium which comprises a TLR3 ligand induces mRNA expression of any of the following genes by at least10-fold when compared to an unstimulated cell population: CXCL9; GBP1; ISG15; SOCS1; MMP3; JAK2 or IRF1. In certain embodiments, the induction medium which comprises a TLR3 ligand induces mRNA expression of any of the following genes by at least 20-fold when compared to an unstimulated cell population: CXCL9; GBP1; ISG15; or SOCS1. In certain embodiments, the induction medium which comprises a TLR3 ligand induces mRNA expression of CXCL9 by at least 100 fold when compared to an unstimulated cell population.

In certain embodiments, the induction medium which comprises a TLR4 ligand induces mRNA expression of *TNFSF10 (TRAIL)*at least 2-fold, 10-fold, 100-fold or 1000 fold when compared to an unstimulated cell population.

The invention also provides an ex-vivo method for inducing, activating or priming a population of mesenchymal stem cells, comprising: (a) providing a population of mesenchymal stem cells; (b) providing an induction medium as disclosed herein; (c) contacting the stem cells with the induction medium; and (d) culturing the stem cells under appropriate conditions.

In certain embodiments, the ex-vivo method for inducing, activating or priming a population of mesenchymal stem cells disclosed herein induces or reduces expression of certain genes which can be measured by methods known to one skilled in the art including, but not limited to: PCR; qPCR; qRT-PCR; semi quantitative RT-PCR; digital PCR; northern blot; mRNA-SEQ; microarrays; and others. In certain embodiments, the induction medium disclosed herein increases or decreases protein levels which can be measured by methods known to one skilled in the art including, but not limited to: antibody based assays; enzyme-linked immunosorbent assay (ELISA); immuno or western blot; flow cytometry, mass spectrometry and others. In certain embodiments, the ex-vivo method for inducing, activating or priming a population of mesenchymal stem cells disclosed herein induces activation or attenuation of cell signaling pathways which can be measured by method known to skilled in the art including, but no limited to: kinase assay; protein phosphorylation/dephosphorylation measurements; protein ubiquitination/deubiquitination measurements; protein acetylation/deacetylation measurements; protein degradation/stability measurements; measurements of second messengers such as calcium or diacylglycerol; or monitoring of cleavage from an inactive to active form.

In certain embodiments, the ex-vivo method for inducing, activating or priming a population of mesenchymal stem cells disclosed herein leads to measurable changes in the gene expression, protein levels, or cell signaling pathways, of a stem cell population. In certain embodiments, the change is an increase in gene expression, protein levels or cell signaling. In certain embodiments, the change is any statistically significant change as measured between an unstimulated or control sample, and a stimulated or test sample. In certain embodiments, the change between an unstimulated or control sample, and a stimulated or test sample, is an increase of at least 2-fold, 3-fold, 4-fold, 5-fold, 6-fold, 7-fold, 8-fold, 9-fold, 10-fold or more. In certain embodiments, the change between an unstimulated or control sample, and a stimulated or test sample, is an increase of at least 100-fold or more. In certain embodiments, the change between an unstimulated or control sample, and a stimulated or test sample, is a decrease of at least 2-fold, 3-fold, 4-fold, 5-fold, 6-fold, 7-fold, 8-fold, 9-fold, 10-fold or more. In certain embodiments, the change between an unstimulated or control sample, and a stimulated or test sample, is a decrease of at least 100-fold or more.

In certain embodiments, the ex-vivo method for inducing, activating or priming a population of mesenchymal stem cells disclosed herein which comprises a TLR3 ligand induces mRNA expression of any of the following genes by at least 2-fold when compared to an unstimulated cell population: CXCL9; EGFR; IRF1; A2M; FAS; IL2RG; MMP3; GBP1; ISG15; FCGR1; NFKB1; NOS2A; USF1; YY1; JAK2; STA2, STAT4; STATS; SOCS1; or IRF1. In certain embodiments, the ex-vivo method for inducing, activating or priming a population of mesenchymal stem cells disclosed herein which comprises a TLR3 ligand reduces mRNA expression of any of the following genes by at least 2-fold when compared to an unstimulated cell population: EPOR; F2R; STAM; PDGFRA; PIAS2; MYC; SH2B1; or CSF2RB. In certain embodiments, the ex-vivo method for inducing, activating or priming a population of mesenchymal stem cells disclosed herein which comprises a TLR3 ligand induces mRNA expression of any of the following genes by at least10-fold when compared to an unstimulated cell population: CXCL9; GBP1; ISG15; SOCS1; MMP3; JAK2 or IRF1. In certain embodiments, the ex-vivo method for inducing, activating or priming a population of mesenchymal stem cells disclosed herein which comprises a TLR3 ligand induces mRNA expression of any of the following genes by at least 20-fold when compared to an unstimulated cell population: CXCL9; GBP1; ISG15; or SOCS1. In certain embodiments, the ex-vivo method for inducing, activating or priming a population of mesenchymal stem cells disclosed herein which comprises a TLR3 ligand induces mRNA expression of CXCL9 by at least 100 fold when compared to an unstimulated cell population.

In certain embodiments, the ex-vivo method for inducing, activating or priming a population of mesenchymal stem cells disclosed herein which comprises a TLR4 ligand induces mRNA expression of *TNFSF 10 (TRAIL)* at least 2-fold, 10-fold, 100-fold or 1000 fold when compared to an unstimulated cell population.

The invention also provides a method cellular therapy, comprising: (a) providing a population of mesenchymal stem cells; (b) providing an induction medium of the invention; (c) contacting the stem cell population with the induction medium; and (d) culturing the cells under appropriate conditions.

The methods of the invention may comprise culturing the cells in contact with a solid surface as described elsewhere herein. For example, the invention provides a method comprising: (a) providing a population of mesenchymal stem cells; (b) providing an induction medium as disclosed herein; (c) contacting the stem cells with the induction medium; and (d) culturing the cells under appropriate conditions and in contact with a solid surface. The invention also provides the use of an induction medium as disclosed herein and a solid surface to expand a population of mesenchymal stem cells. The mesenchymal stem cells may adhere, attach or be seeded onto said support. Typically, the cells are plated at a desired density such as between about 100 cells/cm2 to about 100,000 cells/cm2 (such as about 500 cells/cm2 to about 50,000 cells/cm2, or, more particularly, between about 1,000 cells/cm2 to about 20,000 cells/cm2) prior to inducing, activating or priming of the stem cells. In a particular embodiment, the cell density is between 200-10,000 cells/cm2.

It will be appreciated that the steps of the methods disclosed herein can be performed in any suitable order or at the same time, as appropriate, and need not be performed in the order in which they are listed. For example, in the above method the step of providing a population of mesenchymal stem cells may be performed before, after or at the same time as, the step of providing an induction medium.

The methods and uses of the invention may involve any induction medium or supplement as described herein. Accordingly, in some embodiments, the methods of the invention may be serum and/or serum replacement-free methods. In some embodiments, the methods of the invention may be used to induce cells in the absence of contact with a layer of feeder cells.

The preferred methods and uses of the invention are for the inducing, activating or priming of the population of mesenchymal stem cells to occur once the cells have been expanded and prior to being cryopreserved and used in cell-based therapy.

It is preferred that said stem cell population is of adult origin, and it is further preferred that said cells are a mesenchymal stem cell population, as in bone marrow-derived or adipose tissue-derived cells.

Conditions for the culture of stem cells are known to the person skilled in the art. It is preferred that the culture is carried out in the presence of a solid support suitable for the adherence of mesenchymal stem cells.

Said method of manufacture may optionally further comprise the steps of:
(a) passaging the cells into a culture medium as disclosed herein; (b) further culturing the cells under appropriate conditions and (c) inducing, activating or priming the cells.

It has been shown that ex vivo expansion of the MSC without inducing differentiation can be accomplished for extended time periods for example by using specially screened lots of suitable serum (such as fetal bovine serum or human serum). Methods for measuring viability and yield are known in the art (*e*.*g*., trypan blue exclusion).

Any of the steps and procedures for isolating the cells of the cell population of the invention can be performed manually, if desired. Alternatively, the process of isolating such cells can be facilitated and/or automated through one or more suitable devices, examples of which are known in the art.

Practice of the invention may be performed using any suitable cell culture vessel as a support. Cell culture vessels of various shapes and sizes (e.g. flasks, single or multi-well plates, single or multi-well dishes, bottles, jars, vials, bags, bioreactors) and constructed from various different materials (e.g. plastic, glass) are known in the art. A suitable cell culture vessel can readily be selected by the skilled person.

The invention also provides a culture-medium induction supplement that can be used to produce a culture induction medium as disclosed here. A "culture-medium induction supplement' is a mixture of ingredients that cannot itself support mesenchymal stem cells, but which enables or improves mesenchymal stem cell culture when combined with other cell culture-medium ingredients. The supplement can therefore be used to produce a functional cell culture medium of the invention by combining it with other cell culture ingredients to produce an appropriate medium formulation. The use of culture medium supplements is well known in the art. The invention provides a culture-medium induction supplement that comprises adding a TLR ligand or TLR-ligand inducer in combination with erythropoietin (EPO) and with exposure to hypoxia or hypoxia mimetic (cobalt chloride or desferrioxamine). The supplement may contain any ligands disclosed herein. The supplement may also contain one or more additional cell culture ingredients, e.g. one or more cell culture ingredients selected from the group consisting of amino acids, vitamins, inorganic salts, trace elements, carbon energy sources and buffers.

A culture-medium induction supplement may be a concentrated liquid supplement (*e.g*., a 2x to 250x concentrated liquid supplement) or may be a dry supplement. Both liquid and dry types of supplements are well known in the art. A supplement may be lyophilized.

A culture-medium induction supplement of the invention will typically be sterilized prior to use to prevent contamination, e.g., by ultraviolet light, heating, irradiation or filtration. A culture-medium induction supplement may be frozen (e.g. at -20°C or -80°C) for storage or transport.

The invention also provides a hermetically-sealed vessel containing a culture medium supplement of the invention. Hermetically-sealed vessels may be preferred for transport or storage of the culture medium supplements disclosed herein, to prevent contamination. The vessel may be any suitable vessel, such as a bioreactor, a flask, a plate, a bottle, a jar, a vial, or a bag.

A variety of substances have been used as surfaces for adherent stem cell culture, and an appropriate material can readily be selected by the skilled person. Preferably, the solid surface comprises plastic but may alternatively comprise of glass, extracellular matrix. The surface may be planar, tubular, or in the form of a scaffold, bead or fiber.

The compositions of the invention may comprise serum, or may be serum-free and/or serum-replacement free, as described elsewhere herein.

Mesenchymal stem cells for use in the invention can be obtained using well-known methods (see below). It is envisaged that various types of mesenchymal stem cells may be used in conjunction with the invention, whether obtained from embryonic, fetal, or adult tissue but are preferably derived from adult tissue sources.

The induction medium disclosed herein may be used to culture mammalian stem cells, particularly human adult stem cells. Human adult stem cells that may be used in conjunction with the invention are preferably mesenchymal stem cells. Mouse or primate stem cells may also be used. In preferred embodiments, the stem cells are human bone marrow-derived stem cells (MSC).

Mesenchymal stem cells may be identified by their ability to differentiate into cells of all three germ layers e.g. by determining the ability of the cells to differentiate into cells showing detectable expression of markers specific for all three germ layers. References in the singular (e.g. to "a cell" and equivalent references) encompass the plural (e.g. "cells") unless the context requires otherwise.

The induction medium of the invention can be used to induce, activate or prime a population of mesenchymal stem cells. Accordingly, the invention provides the use of any induction medium as disclosed herein for inducing, activating or priming a population of mesenchymal stem cells into discrete uniform phenotypes for cell-based therapy. These discrete and uniform phenotypes can be an anti-inflammatory MSC phenotype (MSC2), and a uniform and discrete pro-immune anti-tumor MSC phenotype (MSC1).

The preferred method of induction for a uniform and discrete anti-inflammatory MSC phenotype (MSC2) is incubation of the MSC with a culture medium containing a Toll-like receptor-3 (TLR3) ligand such as polyinosinic:polycytidylic acid (or poly(I:C); 1 pg/mL) in combination with erythropoietin (1mU/mL or 5ng/mL) and with exposure to hypoxia (1% oxygen) or hypoxia mimetic (cobalt chloride or desferrioxamine, either at 200µM) for 1 hour upon 70-90% confluent growth.

The preferred method of induction for a uniform and discrete pro-immune anti-tumor MSC phenotype (MSC1) is incubation of the MSC with a culture medium containing a Toll-like receptor-4 (TLR4) ligand such as lipopolysaccharide (LPS, endotoxin at 10 ng/mL) in combination with erythropoietin(1mU/mL or 5ng/mL) and with exposure to hypoxia (1% oxygen) or hypoxia mimetic (cobalt chloride or desferrioxamine, either at 200µM)) for 1 hour upon 70-90% confluent growth.

TLR-ligands in combination with erythropoietin and with exposure to hypoxia or hypoxia mimetic (cobalt chloride or desferrioxamine) are added to fresh culture medium, or as a culture supplement and incubated with the cells for 1 hr. Following this induction step, the MSC are washed twice in culture medium or suitable buffered saline solution without the TLR-ligands to remove cell and culture debris. Without wishing to be bound by theory, short incubation times (< 1 hr) and minimal TLR ligand exposure at the concentrations noted above (or lower) are important for achieving the desired phenotypes and, further, this protocol mimics the gradient of danger signals that endogenous MSCs encounter and respond to at a distance from the site of injury. Once washed, the induced, activated, or primed MSC can be harvested by traditional methods e.g.-trypsin and EDTA for between 5 seconds and 15 minutes at 37°C or with a trypsin substitute (e.g. TrypLE from Invitrogen), collagenase, dispase, accutase or other reagents known to the person skilled in the art. Following cell harvest the primed, activated, or induced MSC can be cryopreserved by standard methods.

The TLR3 agonist or TLR4 agonists may be delivered by incubation, transfection, transduction by carrier molecules, or by other techniques known to those of ordinary skill in the art.

The cells may be incubated with TLR ligand or agonist ligand in combination with erythropoietin (EPO) and with exposure to hypoxia or hypoxia mimetic (cobalt chloride or desferrioxamine) for from about 1 minute to about 480 minutes, from about 5 minutes to about 475 minutes, from about 10 minutes to about 470 minutes, from about 15 minutes to about 400 minutes, from about 20 minutes to about 120 minutes, from about 25 minutes to about 90 minutes, from about 30 minutes to about 80 minutes, from about 35 minutes to about 70 minutes, from about 40 minutes to about 65 minutes, from about 45 minutes to about 60 minutes, from about 55 minutes to about 60 minutes, and preferably about 60 minutes.

### EXAMPLES

### Example 1-Induction of an MSC2 gene expression signature from human primary MSC

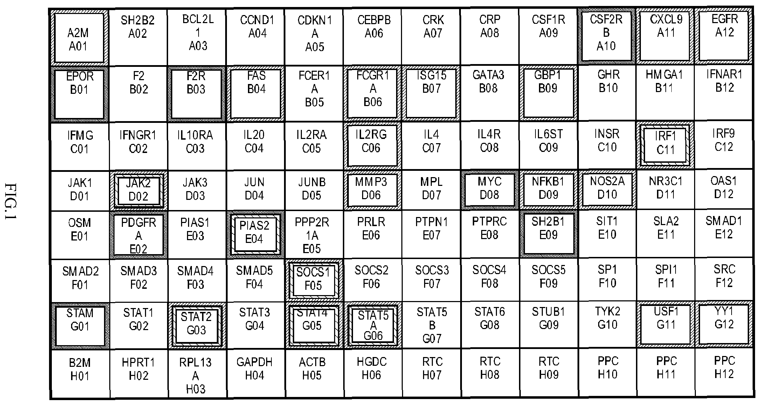

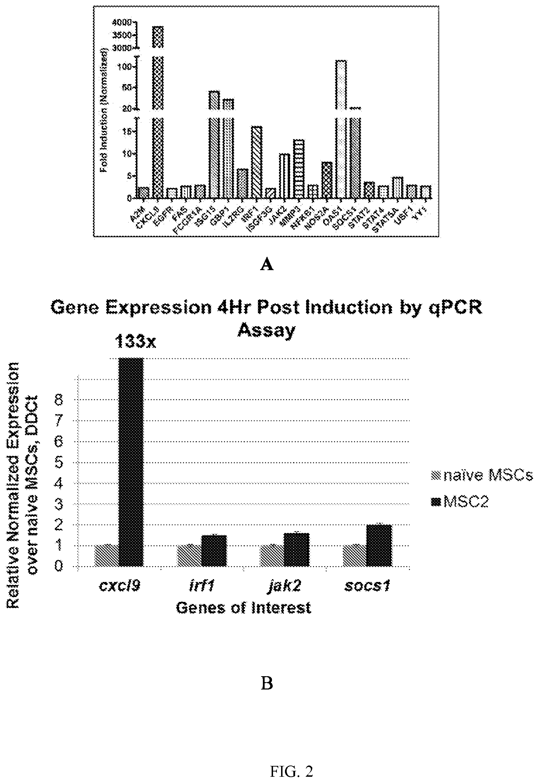

For this experiment primary human MSC were incubated at 37°C and 5% CO2, in serum-free medium containing 2 µg/mL poly(I:C), for 6 hours. Subsequently, cells were washed twice, RNA was extracted using the RNeasy Mini Kit (Qiagen, Valencia, CA), and then treated using the TURBO DNA-free kit (Ambion, Austin, TX). RNA was reverse transcribed and the resulting cDNA was used in the JAK/STAT Signaling Pathway RT2 ProfilerTM PCR Array (SuperArray Bioscience, Frederick, MD) according to the manufacturer's instructions on an iCycler iQ5 Real-Time PCR Detection System (Bio-Rad, Hercules, CA). Raw data from both the untreated and treated groups were analyzed using the GEarray Analyzer software (SuperArray Inc., Bethesda, MD). This Array measures RNA expression levels of 84 different genes in the JAK/STAT signaling pathway. Results are shown in FIG. 1, Genes which show a 2-fold or greater induction are boxed in grey, genes which show a 2-fold or greater reduction are boxed in black, and genes that were induced by greater than two-fold and selected for further validation are depicted in thick boxes (except for PIAS2 which was reduced by greater than 2-fold). FIG. 2 shows further qPCR validation of genes selected in FIG. 1. Validation was performed by qPCR using a SYBR green Master Mix with gene specific primers on the same samples analyzed in FIG. 1.

### Example-2 MSC1 and MSC2 polarization from primary human MSC

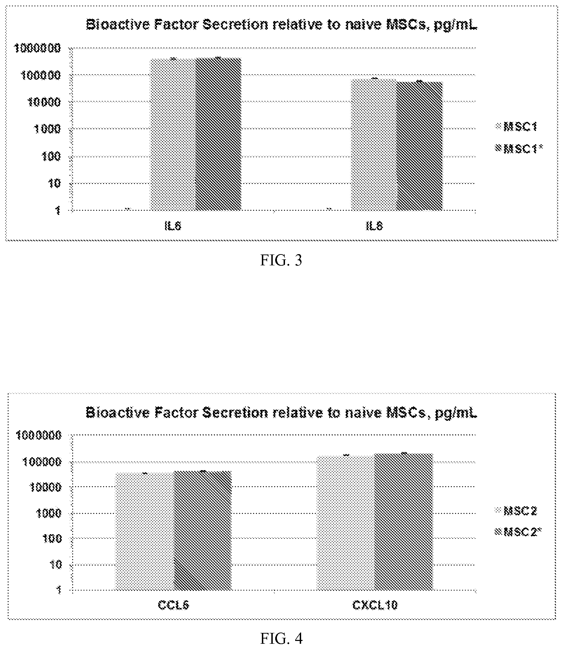

Primary Human MSC donors were polarized to MSC1 or MSC2. Human MSC were polarized using a medium containing 10 ng/mL of LPS in either the absence (MSC1), or presence of 0.5 ng/mL human recombinant erythropoietin and 200 µM Cobalt Chloride (MSC1^{∗}). MSC2 cells were polarized using a medium containing 2 µg/mL of poly(I:C) in either the absence (MSC2), or presence of 0.5 ng/mL human recombinant erythropoietin and 200 µM Cobalt Chloride (MSC2*). The cultured supernatants were harvested and then analyzed for chemokine/cytokine expression by bio-plex as previously described. Briefly, MSCs were plated at a density of 50,000 cells in 24-well plates, allowed to adhere overnight, then primed with TLR agonists for 1 hr as indicated. Conditioned medium was collected after 48 hr and analyzed with Bio-Plex Cytokine Assays (Human Group I & II; Bio-Rad, Hercules, CA) following the manufacturer's instructions. These experiments were performed at least three times on three individual MSC donor pools. MSC1 induction regardless of formula results in significant secretion of cytokine including IL6, and IL8 while (**FIG. 3**), MSC2 induction regardless of formula results in significant secretion of IP10 (CXCL10), and RANTES (CCL5) (**FIG. 4**). Error bars indicate +/- SEM.

### Example 3-MSC1 and MSC2 polarization is consistent across multiple sources of MSC

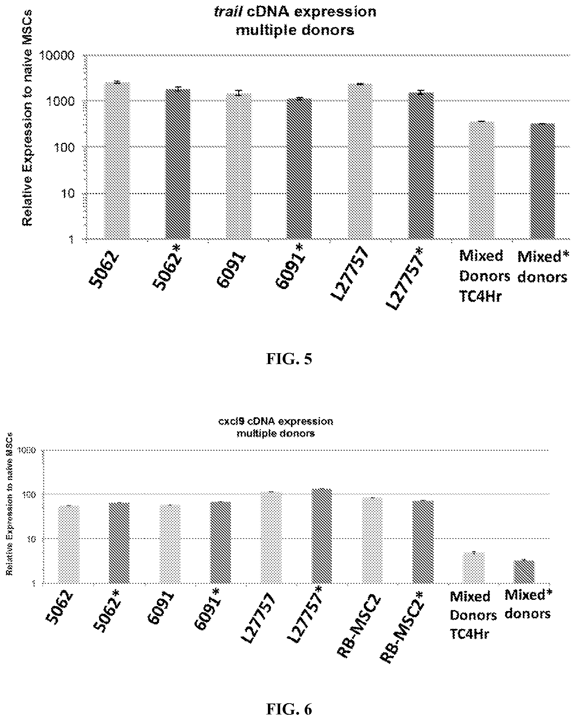

Human MSCs donors from 3 different commercial sources and up to six different donors were polarized to MSC1. Human MSC were polarized using a medium containing 10 ng/mL of LPS in either the absence (MSC1), or presence of 0.5 ng/mL human recombinant erythropoietin and 200 µM Cobalt Chloride (MSC1^{∗}). Total RNA was isolated, purified, and reverse transcribed to cDNA. Quantitative real-time PCR was carried out using SYBR Green Master Mix. Data were analyzed using the quantitative comparative CT method, normalizing target gene expression to the *18S rRNA* housekeeping gene, and shown as fold increase over the untreated control. *Trail* gene expression was significantly increased following MSC1 induction in all donors including mixed ones (**FIG.5**). Error bars represent +/- the standard error of the mean (SEM). The **human cDNA primers used:** *Cxcl9* **Forward**-CTT TCCTGG CTA CTC CAT GTT **Reverse**-GTT GGT CACTGG CTG ATC TAT AA; *Trail* **Forward**-CTT CAC AGT GCT CCT GCA GT **Reverse-**TTA GCC AACTAA AAA GGC CCC; *18SrRNA* **Forward-** GAGGGAGCCTGAGAAACGG, **Reverse**-GTCGGGAGTGGGTAATTTGC **with the protocol: 1:** 95.0°C for 0:30, **2:** 95.0°C for 0:10, **3:** 68.0°C for 0:30, Plate Read, **4:** GOTO 2, 39 more times.

Human MSCs donors from 3 different commercial sources and up to six different donors were polarized to MSC2. Human MSC were polarized using a medium containing 2 µg/mL of poly(I:C) in either the absence (MSC2), or presence of 0.5 ng/mL human recombinant erythropoietin and 200 µM Cobalt Chloride (MSC2*). Total RNA was isolated, purified, and reverse transcribed to cDNA. Quantitative real-time PCR was carried out using SYBR Green Master Mix. Data were analyzed using the quantitative comparative CT method, normalizing target gene expression to the 18S rRNA housekeeping gene, and shown as fold increase over the untreated control. *CXCL9* gene expression was significantly increased following MSC2 induction in all donors including mixed ones (**FIG. 6**). Error bars represent +/- the standard error of the mean (SEM).

### Example 4 -time course of MSC1 and MSC2 polarization.

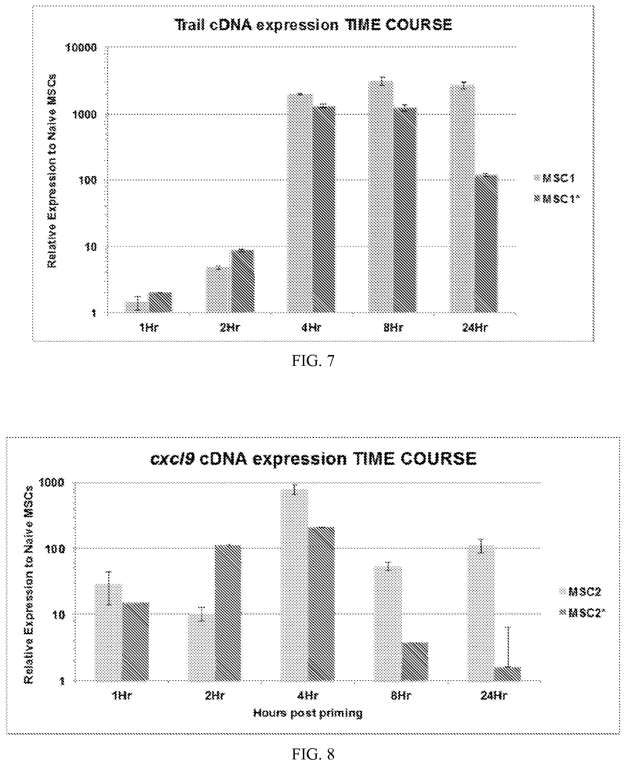

Human MSCs donors were polarized to MSC1. Human MSC were polarized using a medium containing 10 ng/mL of LPS in either the absence (MSC1), or presence of 0.5 ng/mL human recombinant erythropoietin and 200 µM Cobalt Chloride (MSC1^{∗}). Cells were harvested at different times as indicated. Total RNA was isolated, purified, and reverse transcribed to cDNA. Quantitative real-time PCR was carried out using SYBR Green Master Mix. Data were analyzed using the quantitative comparative CT method, normalizing target gene expression to the 18S rRNA housekeeping gene, and shown as fold increase over the untreated control. Trail gene expression was significantly increased 4hrs following MSC1 induction (**FIG. 7**). Error bars represent +/- the standard error of the mean (SEM).

Human MSCs donors were polarized to MSC2. Human MSC were polarized using a medium containing 2 µg/mL ofpoly(I:C) in either the absence (MSC2), or presence of 0.5 ng/mL human recombinant erythropoietin and 200 µM Cobalt Chloride (MSC2*). Cells were harvested at different times as indicated. Total RNA was isolated, purified, and reverse transcribed to cDNA. Quantitative real-time PCR was carried out using SYBR Green Master Mix. Data were analyzed using the quantitative comparative CT method, normalizing target gene expression to the 18S rRNA housekeeping gene, and shown as fold increase over the untreated control. Cxcl9 gene expression was significantly increased 4hrs following MSC2 induction **(****FIG. 8**). Error bars represent +/- the standard error of the mean (SEM).

### Example 5- Bio distribution of MSC1 and MSC2 cells

An experiment to determine the in vivo efficacy of polarized MSC compared to naive MSCs was conducted. For this experiment, human naive MSCs, MSC1 and MSC2 (1 million cells) were administered by IP injection into wild type mice and following 4 hours all organs were harvested. Subsequently extracted RNA was assayed for human GAPDH and compared with mouse GAPDH DNA to determine tissue homing. Results are shown below in Table 1.

| | **Percent recovered cells, 4 hours post administration** | | |
|---|---|---|---|
| | **naïve MSCs** | **MSC1** | **MSC2** |
| **Brain** | **0.10** | **0.3** | **0.4** |
| **Lung** | *54.20* | *3.1* | 4.9 |
| **Heart** | **0.20** | **0.001** | **0.5** |
| **Spleen** | *25.50* | 46.7 | 82.9 |
| **Kidney** | **20.60** | **26.9** | **2.6** |
| **Liver** | **1.70** | **22.2** | **8.9** |
| **Intestine** | **0.60** | **0.001** | **0** |
| **Lymph Nodes** | **0.00** | **0.2** | **0** |

### Example 6-Incubatation with erythropoietin and cobalt chloride during MSC polarization increases migration, proliferation/viability of MSC1 and MSC2 cells

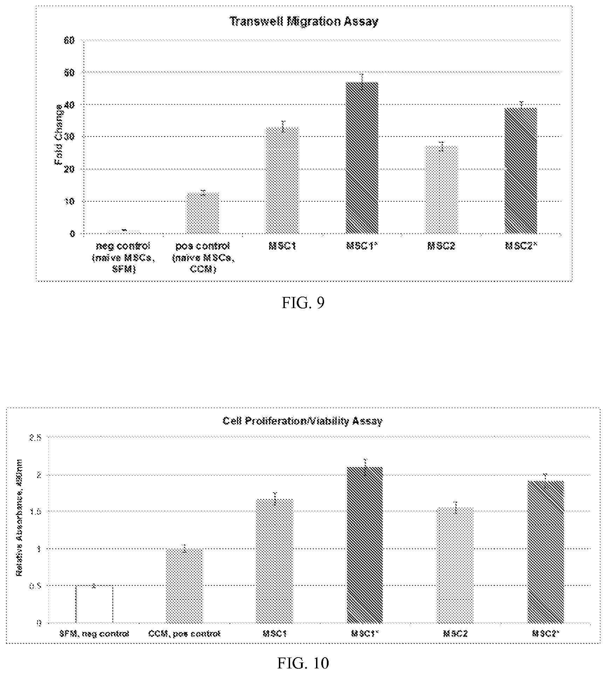

To determine the migration capacity of MSC cultured with or without erythropoietin and hypoxia, human naive MSC cells, and either MSC1 or MSC2 polarized cells were added to individual transwell inserts (8uM pores, 50,000 cells/insert). MSC1 cells were polarized using a medium containing 10 ng/mL of LPS in either the absence (MSC1), or presence of 0.5 ng/mL human recombinant erythropoietin and 200 µM Cobalt Chloride (MSC1^{∗}). MSC2 cells were polarized using a medium containing 2 µg/mL of poly (I:C) in either the absence (MSC2), or presence of 0.5 ng/mL human recombinant erythropoietin and 200 µM Cobalt Chloride (MSC2*). The membranes were lowered into 24-well companion plates containing either negative control serum free medium (SFM) or the positive control, serum containing growth medium (CCM), or the corresponding inducing medium as indicated. Photomicrographs were taken following 16 hours of incubation with a Nikon Eclipse TE300 inverted fluorescence microscope. **FIG. 9** shows representative data of migrated MSC counted from 4 representative picture quadrants from greater than three independently performed experiments done in triplicate (n =3). Error bars indicate SEM.

A proliferation assay was used to determine the proliferative capacity and viability of MSC cultured with or without erythropoietin and hypoxia. Human MSC were polarized using a medium containing 10 ng/mL of LPS in either the absence (MSC1), or presence of 0.5 ng/mL human recombinant erythropoietin and 200 µM Cobalt Chloride (MSC1^{∗}). MSC2 cells were polarized using a medium containing 2 µg/mL of poly(I:C) in either the absence (MSC2), or presence of 0.5 ng/mL human recombinant erythropoietin and 200 µM Cobalt Chloride (MSC2*). Cells were incubated with the specified medium for 48 hours. Cell proliferation and viability from each sample was measured by CyQUANT assay (Life Technologies, CA) and trypan blue assay respectively. For cell proliferation cells were seeded in 96-well plates at 1×10³ cells in 50 µl per well in triplicate and cultured at 37°C, 5% CO₂. Samples were harvested at 0,24,48,72, and 96 hrs after treatment and processed as described by manufacturer (CyQUANT assay, Life Technologies, CA). Data shown are expressed relative to untreated controls. Cell proliferation assay was performed on at least three separate experiments with each sample repeated along 8 wells of the 96-well plate (n=3). Error bars indicate SEM. Results are shown in **FIG. 10****.**

### Example 7 validation of qPCR assay for CXCL9 and TNFSF10

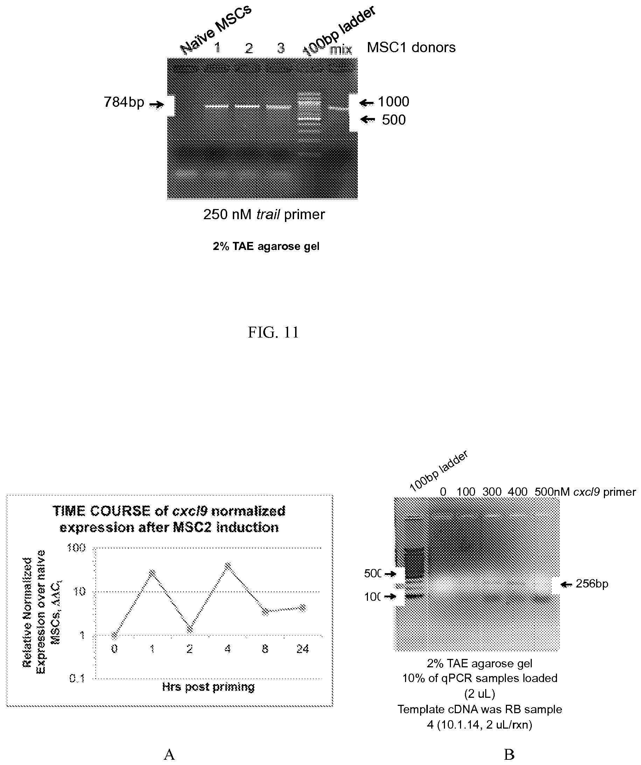

Human MSCs were induced into MSC1. Total RNA was isolated, purified, and reverse transcribed to cDNA. Quantitative real-time PCR was carried out using SYBR Green Master Mix. Data were analyzed using the quantitative comparative CT method, normalizing target gene expression to the 18S rRNA housekeeping gene, and shown as fold increase over the untreated control. Trail gene expression was significantly increased following MSC1 induction. Error bars represent +/- the standard error of the mean (SEM). Primer efficiency and product specificity (**FIG. 11**) were established.

Human MSCs were induced into MSC2. Total RNA was isolated, purified, and reverse transcribed to cDNA. Quantitative real-time PCR was carried out using SYBR Green Master Mix. Data were analyzed using the quantitative comparative CT method, normalizing target gene expression to the 18S rRNA housekeeping gene, and shown as fold increase over the untreated control. Cxcl9 gene expression was significantly increased following MSC2 induction. Error bars represent +/- the standard error of the mean (SEM). Time course of gene expression (FIG. 12A), and product specificity (FIG. 12B) were established.

While this invention has been described in detail with particular reference to its preferred embodiments, the principles and modes of operation of the invention have also been described in this specification. The invention should not be construed as being limited to the particular forms disclosed, which are illustrative rather than restrictive. Modifications, variations, and changes may be made by those skilled in the art without departure from the spirit and scope of the invention as described by the following claims.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### ASPECTS OF THE INVENTION

1. An induction medium for creating an immunologically polarized mesenchymal stem cell population from an unstimulated mesenchymal stem cell population, the induction medium comprising:
   a. a Toll-like receptor 3 (TLR3) ligand,
   b. erythropoietin, and
   c. 0.5-2% oxygen or a hypoxia mimetic,
   wherein the immunologically polarized mesenchymal stem cell population possess anti-inflammatory characteristics marked by expression of anti-inflammatory or immunosuppressive mediators.
2. The induction medium of aspect 1, wherein the Toll-like receptor 3 (TLR3) ligand is poly(I:C).
3. The induction medium of aspect 1, wherein the Toll-like receptor 3 (TLR3) ligand is poly(A:U).
4. The induction medium of aspect 1, wherein erythropoietin is present at a concentration of less than 10 ng/mL.
5. The induction medium of aspect 1, wherein the hypoxia mimetic is cobalt chloride.
6. The induction medium of aspect 5, wherein the cobalt chloride is present at a concentration of between 5 µM and 500 µM.
7. The induction medium of aspect 1, further comprising interleukin 4 (IL-4).
8. The induction medium of aspect 1, further comprising interleukin 13 (IL-13).
9. The induction medium of aspect 1, which does not comprise serum of human or animal origin.
10. The induction medium of aspect 1, which is a concentrated solution.
11. A mesenchymal stem cell population treated with the induction medium of aspect 1.
12. A human mesenchymal stem cell population treated with the induction medium of aspect 1.
13. A canine, feline, or equine mesenchymal stem cell population treated with the induction medium of aspect 1.
14. A mesenchymal stem cell population treated with the induction medium of aspect 1, wherein the mesenchymal stem cells were derived from a pluripotent stem cell.
15. A mesenchymal stem cell population treated with the induction medium of aspect 1, wherein the cells are marked by increased expression of *CXCL9* mRNA compared to an unstimulated mesenchymal stem cell population.
16. A mesenchymal stem cell population treated with the induction medium of aspect 1, wherein the cells are marked by increased expression of *OAS1* mRNA compared to an unstimulated mesenchymal stem cell population.
17. A mesenchymal stem cell population treated with the induction medium of aspect 1, wherein the cells are marked by increased expression of *ISG15* mRNA compared to an unstimulated mesenchymal stem cell population.
18. A composition for the treatment of a disease comprising a mesenchymal stem cell population treated with the induction medium of aspect 1, wherein the disease is an inflammatory or autoimmune disorder.
19. The composition of aspect 18, wherein the inflammatory or autoimmune disorder is rheumatoid arthritis.
20. The composition of aspect 18, wherein the inflammatory or autoimmune disorder is an inflammatory bowel disease.
21. The composition of aspect 18, wherein the inflammatory or autoimmune disorder is Acute Optic Neuritis.
22. The composition of aspect 18, wherein the inflammatory or autoimmune disorder is Krabbe disease.
23. The composition of aspect 18, wherein the inflammatory or autoimmune disorder is Diabetic Retinopathy.
24. The composition of aspect 18, wherein the inflammatory or autoimmune disorder is Crohn's Disease.
25. The composition of aspect 18, wherein the inflammatory or autoimmune disorder is Acute Lung Injury.
26. An induction medium for creating an immunologically polarized mesenchymal stem cell population from an unstimulated mesenchymal stem cell population, the induction medium comprising:
   a. a Toll-like receptor 4 (TLR4) ligand,
   b. erythropoietin, and
   c. 0.5-2% oxygen or a hypoxia mimetic,
   wherein the immunologically polarized mesenchymal stem cell population possess pro-inflammatory characteristics marked by expression of pro-inflammatory mediators.
27. The induction medium of aspect 26, wherein the Toll-like receptor 4 (TLR4) ligand is lipopolysaccharide (LPS).
28. The induction medium of aspect 26, wherein the Toll-like receptor 4 (TLR4) ligand is an aminoalkyl glucosaminide 4-phosphate.
29. The induction medium of aspect 26, wherein erythropoietin is present at a concentration of less than 10 ng/mL.
30. The induction medium of aspect 26, wherein the hypoxia mimetic is cobalt chloride.
31. The induction medium of aspect 30, wherein the cobalt chloride is present at a concentration of between 5 µM and 500 µM.
32. The induction medium of aspect 26, further comprising an interferon.
33. The induction medium of aspect 26, further comprising tumor necrosis factor alpha (TNFα).
34. The induction medium of aspect 26, which does not comprise serum of human or animal origin.
35. The induction medium of aspect 26, which is a concentrated solution.
36. A mesenchymal stem cell population treated with the induction medium of aspect 26.
37. A human mesenchymal stem cell population treated with the induction medium of aspect 26.
38. A canine, feline, or equine mesenchymal stem cell population treated with the induction medium of aspect 26.
39. A mesenchymal stem cell population treated with the induction medium of aspect 26, wherein the mesenchymal stem cells were derived from a pluripotent stem cell.
40. A mesenchymal stem cell population treated with the induction medium of aspect 26,
   wherein the cells are marked by increased expression of *TNFSF10 (TRAIL)* mRNA compared to an unstimulated mesenchymal stem cell population.
41. A composition for the treatment of a disease comprising a mesenchymal stem cell population treated with the induction medium of aspect 26, wherein the disease is a cancer.
42. A composition for the treatment of a disease comprising a mesenchymal stem cell population treated with the induction medium of aspect 26, wherein the disease is ovarian cancer.
43. A composition for the treatment of a disease comprising a mesenchymal stem cell population treated with the induction medium of aspect 26, wherein the disease is uveal melanoma.
44. A composition for the treatment of a disease comprising a mesenchymal stem cell population treated with the induction medium of aspect 26, wherein the disease is a viral condition.
45. A composition for the treatment of a disease comprising a mesenchymal stem cell population treated with the induction medium of aspect 26, wherein the disease is a bacterial infection.
46. An induction medium for creating an immunologically polarized mesenchymal stem cell population from an unstimulated mesenchymal stem cell population, the induction medium comprising:
   a. Poly(I:C) at a concentration of between 0.1 µg/mL and 100 µg/mL,
   b. erythropoietin at a concentration of less than 10 ng/mL, and
   c. cobalt chloride at a concentration of between 5 µM and 500 µM,
   wherein the immunologically polarized mesenchymal stem cell population possess anti-inflammatory characteristics and is marked by increased expression of *CXCL9, OAS1* and *ISG15* mRNA compared to an unstimulated mesenchymal stem cell population.
47. An induction medium for creating an immunologically polarized mesenchymal stem cell population from an unstimulated mesenchymal stem cell population, the induction medium comprising:
   a. LPS at a concentration of between 0.1 ng/mL and 1 µg/mL,
   b. erythropoietin at a concentration of less than 10 ng/mL, and
   c. cobalt chloride at a concentration of between 5 µM and 500 µM,
   wherein the immunologically polarized mesenchymal stem cell population possess pro inflammatory characteristics and is marked by increased expression of *TNFSF10 (TRAIL)* compared to an unstimulated mesenchymal stem cell population.
48. A method for creating an immunologically polarized mesenchymal stem cell population from an unstimulated mesenchymal stem cell population, the method comprising: contacting an unstimulated mesenchymal stem cell population with a composition comprising; a Toll-like receptor 3 (TLR3) ligand, erythropoietin, and exposure to hypoxia or a hypoxia mimetic, wherein the immunologically polarized mesenchymal stem cell population possess anti-inflammatory characteristics marked by expression of anti-inflammatory or immunosuppressive mediators.
49. The method of aspect 48, wherein the Toll-like receptor 3 (TLR3) ligand is poly(I:C).
50. The method of aspect 48, wherein the Toll-like receptor 3 (TLR3) ligand is poly(A:U).
51. The method of aspect 48, wherein erythropoietin is present at a concentration of less than 10 ng/mL.
52. The method of aspect 48, wherein the hypoxia mimetic is cobalt chloride.
53. The method of aspect 52, wherein the cobalt chloride is present at a concentration of between 5 µM and 500 µM.
54. The method of aspect 48, wherein the composition further comprises interleukin 4 (IL 4).
55. The method of aspect 48, wherein the composition further comprises interleukin 13 (IL-13).
56. The method of aspect 48, wherein the composition does not comprise serum of human or animal origin.
57. The method of aspect 48, wherein the composition is a concentrated solution.
58. The method of aspect 48, wherein the unstimulated mesenchymal stem cell population is contacted with a Toll-like receptor 3 (TLR3) ligand, erythropoietin, and hypoxia or a hypoxia mimetic simultaneously.
59. The method of aspect 48, wherein the composition is in contact with the unstimulated mesenchymal stem cell population for at least 30 minutes, but less than 8 hours.
60. The method of aspect 48, further comprising monitoring expression of *CXCL9* at either the RNA or protein level.
61. The method of aspect 48, further comprising monitoring expression of *OAS1* at either the RNA or protein level.
62. The method of aspect 48, further comprising monitoring expression of *ISG15* at either the RNA or protein level.
63. A mesenchymal stem cell population treated by the method of aspect 48.
64. A human mesenchymal stem cell population treated by the method of aspect 48.
65. A canine, feline, or equine mesenchymal stem cell population treated by the method of aspect 48.
66. A mesenchymal stem cell population treated by the method of aspect 48, wherein the mesenchymal stem cells were derived from a pluripotent stem cell.
67. A mesenchymal stem cell population treated by the method of aspect 48, wherein the cells are marked by increased expression of *CXCL9* mRNA compared to an unstimulated mesenchymal stem cell population.
68. A mesenchymal stem cell population treated by the method of aspect 48, wherein the cells are marked by increased expression of *OAS1* mRNA compared to an unstimulated mesenchymal stem cell population.
69. A mesenchymal stem cell population treated by the method of aspect 48, wherein the cells are marked by increased expression of *ISG15* mRNA compared to an unstimulated mesenchymal stem cell population.
70. A method for the treatment of a disease comprising a mesenchymal stem cell population treated by the method of aspect 48, wherein the disease is an inflammatory or autoimmune disorder.
71. The method of aspect 70, wherein the inflammatory or autoimmune disorder is rheumatoid arthritis.
72. The method of aspect 70, wherein the inflammatory or autoimmune disorder is an inflammatory bowel disease.
73. The method of aspect 70, wherein the inflammatory or autoimmune disorder is rheumatoid arthritis.
74. The method of aspect 70, wherein the inflammatory or autoimmune disorder Acute Optic Neuritis.
75. The method of aspect 70, wherein the inflammatory or autoimmune disorder is Krabbe disease.
76. The method of aspect 70, wherein the inflammatory or autoimmune disorder is Diabetic Retinopathy.
77. The method of aspect 70, wherein the inflammatory or autoimmune disorder is Crohn's Disease.
78. The method of aspect 70, wherein the inflammatory or autoimmune disorder is Acute Lung Injury.
79. A method for creating an immunologically polarized mesenchymal stem cell population from an unstimulated mesenchymal stem cell population, the method comprising: contacting an unstimulated mesenchymal stem cell population with a composition comprising; a Toll-like receptor 4 (TLR4) ligand, erythropoietin, and exposure to hypoxia or a hypoxia mimetic, wherein the immunologically polarized mesenchymal stem cell population possess pro-inflammatory characteristics marked by expression of pro-inflammatory mediators.
80. The method of aspect 79, wherein the Toll-like receptor 4 (TLR4) ligand is lipopolysaccharide (LPS).
81. The method of aspect 79, wherein the Toll-like receptor 4 (TLR4) ligand is an aminoalkyl glucosaminide 4-phosphate.
82. The method of aspect 79, wherein erythropoietin is present at a concentration of less than 10 ng/mL.
83. The method of aspect 79, wherein the hypoxia mimetic is cobalt chloride.
84. The method of aspect 83, wherein the cobalt chloride is present at a concentration of between 5 µM and 500 µM.
85. The method of aspect 79, wherein the composition further comprises an interferon.
86. The method of aspect 79, wherein the composition further comprises tumor necrosis factor alpha (TNFα).
87. The method of aspect 79, wherein the composition does not comprise serum of human or animal origin.
88. The method of aspect 79, wherein the composition is a concentrated solution.
89. The method of aspect 79, wherein the unstimulated mesenchymal stem cell population is contacted with a Toll-like receptor 4 (TLR4) ligand, erythropoietin, and hypoxia or a hypoxia mimetic simultaneously.
90. The method of aspect 79, wherein the composition is in contact with the unstimulated mesenchymal stem cell population for at least 30 minutes, but less than 8 hours.
91. The method of aspect 79, further comprising monitoring expression of *TNFSF10 (TRAIL)* at either the RNA or protein level.
92. A mesenchymal stem cell population treated by the method of aspect 79.
93. A human mesenchymal stem cell population treated by the method of aspect 79.
94. A canine, feline or equine mesenchymal stem cell population treated by the method of aspect 79.
95. A mesenchymal stem cell population treated by the method of aspect 79, wherein the mesenchymal stem cells were derived from a pluripotent stem cell.
96. A mesenchymal stem cell population treated by the method of aspect 79, wherein the cells are marked by increased expression of *TNFSF10 (TRAIL)* mRNA compared to an unstimulated mesenchymal stem cell population.
97. A method for the treatment of a disease comprising a mesenchymal stem cell population treated by the method of aspect 79, wherein the disease is a cancer.
98. A method for the treatment of a disease comprising a mesenchymal stem cell population treated by the method of aspect 97, wherein the cancer is ovarian cancer.
99. A method for the treatment of a disease comprising a mesenchymal stem cell population treated by the method of aspect 97, wherein the cancer is uveal melanoma.
100. A method for the treatment of a disease comprising a mesenchymal stem cell population treated by the method of aspect 79, wherein the disease is a viral condition.
101. A method for the treatment of a disease comprising a mesenchymal stem cell population treated by the method of aspect 79, wherein the disease is a bacterial infection.

## Claims

1. An induction medium for creating an immunologically polarized mesenchymal stem cell population from an unstimulated mesenchymal stem cell population, the induction medium comprising:
(a) a Toll-like receptor 3 (TLR3) ligand, and
(b) erythropoietin;
wherein the immunologically polarized mesenchymal stem cell population possesses anti-inflammatory characteristics marked by expression of anti-inflammatory or immunosuppressive mediators.

2. The induction medium of claim 1, wherein the Toll-like receptor 3 (TLR3) ligand is poly(I:C) or poly(A:U).

3. The induction medium of either of claims 1 or 2, wherein erythropoietin is present at a concentration of less than 10 ng/ml.

4. The induction medium of any of claims 1-3 further comprising interleukin 4 (IL-4) and/or interleukin 13 (IL-13).

5. The induction medium of any of claims 1-4, which does not comprise serum of human or animal origin.

6. An induction medium for creating an immunologically polarized mesenchymal stem cell population from an unstimulated mesenchymal stem cell population, the induction medium comprising:
(a) poly(I:C) at a concentration of between 0.1µg/ml and 100 µg/ml, and
(b) erythropoietin at a concentration of less than 10 ng/ml; and
wherein the immunologically polarized mesenchymal stem cell population possesses anti-inflammatory characteristics and is marked by expression of anti-inflammatory or immunosuppressive mediators.

7. The induction medium of any of claims 1-6, which is a concentrated solution.

8. A composition comprising:
(a) the induction medium of any of claims 1-6; and
(b) a population of mesenchymal stem cells.

9. The composition of claim 8, wherein the mesenchymal stem cells are human mesenchymal stem cells.

10. A method comprising culturing the composition of either of claims 8 or 9 under hypoxic conditions of less than 2% oxygen.

11. A method for making an immunologically polarized mesenchymal stem cell population from an unstimulated mesenchymal stem cell population, the method comprising
culturing the unstimulated mesenchymal stem cell population in the induction medium of any of claims 1-6, under hypoxic conditions of less than 2% oxygen;
wherein the immunologically polarized mesenchymal stem cell population possesses anti-inflammatory characteristics marked by expression of anti-inflammatory or immunosuppressive mediators.

12. The method of claim 11, wherein the mesenchymal stem cells are human mesenchymal stem cells.

13. An immunologically polarized mesenchymal stem cell population made by the method of either of claims 11 or 12; wherein the immunologically polarized mesenchymal stem cells are marked by increased expression of CXCL9 mRNA compared to the unstimulated mesenchymal stem cell population.

14. The immunologically polarized mesenchymal stem cell population of claim 13,
wherein the mesenchymal stem cells are human mesenchymal stem cells.

15. The immunologically polarized mesenchymal stem cell population of either of claims 13 or 14, wherein the immunologically polarized mesenchymal stem cell population is for use in a method of treating an inflammatory or autoimmune disorder such as rheumatoid arthritis, inflammatory bowel disease, Crohn's disease, acute optic neuritis, Krabbe disease, diabetic retinopathy, or acute lung injury.
